# FASCICULE DE BREVET EUROPEEN

(11) **EP 1 092 717 B1**
(45) Date de publication et mention de la délivrance du brevet: **08.01.2003**
(21) Numéro de dépôt: 00402832.0
(22) Date de dépôt: 13.10.2000
(51) Int. Cl.: C07D 471/14, C07D 498/14, A61K 31/4355, A61K 31/55, A61K 31/5365, A61P 25/00

(54) **Dérivés polycycliques azaindolinques, leur procédé de préparation et les compositions pharmaceutiques qui les contiennent**
Polycyclische Azaindolderivate, Verfahren zu ihrer Herstellung und diese enthaltende pharmazeutische Zubereitungen
Polycyclic azaindole derivatives, process for their preparation and pharmaceutical compositions containing them

(30) Priorité: 15.10.1999 FR 9912900
(43) Date de publication de la demande: 18.04.2001
(73) Titulaire: LES LABORATOIRES SERVIER, 92200 Neuilly sur Seine (FR)
(72) Inventeur: Guillaumet, Gérald, 45650 Saint Jean le Blanc (FR); Viaud, Marie-Claude, 37000 Tours (FR); Van de Poel, Hervé, 18500 Marmagne (FR); Delagrange, Philippe, 92130 Issy les Moulineaux (FR); Bennejean, Caroline, 94220 Charenton le Pont (FR); Renard, Pierre, 78150 Le Chesnay (FR)

(56) Documents cités:
- WO-A-00/44753
- WO-A-94/07859

## Description

L'invention concerne de nouveaux dérivés polycycliques azaindoliques, leur procédé de préparation et les compositions pharmaceutiques qui les contiennent.

L'art antérieur décrit des composés polycyliques azaindoliques utiles en synthèse (Heterocycles, 41 (9), 1995, pp. 1987-98 ; Tetrahedron Letters, 26 (10), 1985, pp. 1295-6) ou en tant qu'agents antitumoraux et antiviraux (Tetrahedron, 50 (13), 1994, pp. 3987-92).

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272) et analgésiques (Pharmacopsychiat., 1987, 20, pp. 222-223) ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986, 24, pp. 359-364), et dans le traitement de l'obésité (International Journal of Eating Disorders, 1996, 20 (4), pp. 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sites de liaison mélatoninergiques.

Plus particulièrement, la présente invention concerne les composés de formule (I) : 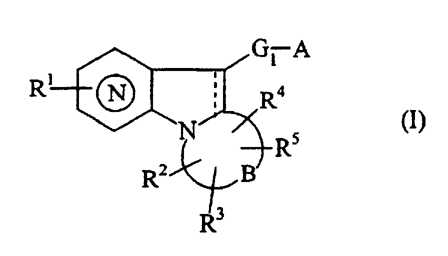 dans laquelle :
- la notation 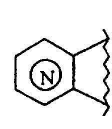 représente les groupements 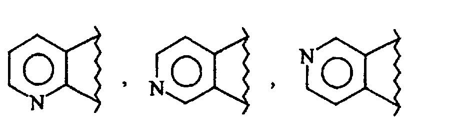 ou 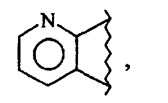
- R¹ représente un atome d'halogène ou un groupement -R, -OR, -S(O)ₙR, -NRR', 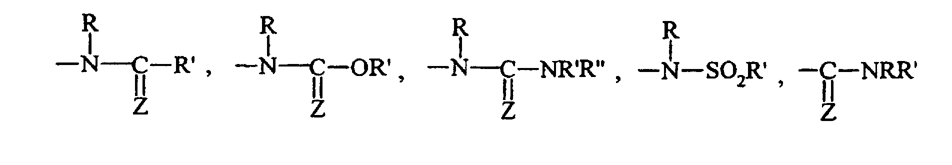 ou -SO₂NRR' (où n vaut 0, 1 ou 2, Z représente un atome de soufre ou d'oxygène, et R, R' et R", identiques ou différents, représentent un atome d'hydrogène ou un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, cycloalkyle (C₃-C₈) substitué ou non, cycloalkyl (C₃-C₈) alkyle (C₁-C₆) linéaire ou ramifié substitué ou non, aryle, arylalkyle (C₁-C₆) linéaire ou ramifié, hétéroaryle ou hétéroarylalkyle (C₁-C₆) linéaire ou ramifié,
   (R et R') et (R' et R") pouvant de plus former ensemble avec l'atome d'azote qui les porte un groupement morpholinyle, piperidinyle, piperazinyle ou pyrrolidinyle, ces groupements étant non substitués ou substitués),
- G₁ représente une chaîne alkylène comportant de 1 à 4 atomes de carbone éventuellement substituée par un groupement R, OR, COR ou COOR (où R est tel que défini précédemment),
   ou G₁ représente une chaîne alkylène comportant de 1 à 4 atomes de carbone dans laquelle un des groupements CH₂ peut être remplacé par un groupement cycloalkylène (C₃-C₈),
- A représente un groupement 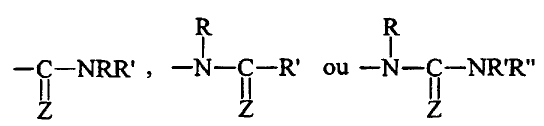 dans lesquels R, R', R" et Z sont tels que définis précédemment,
- B forme avec l'atome d'azote et l'atome de carbone auxquels il est rattaché un cycle comportant de 5 à 8 chaînons pouvant contenir une ou plusieurs insaturations et pouvant contenir, en plus de l'atome d'azote, un hétéroatome supplémentaire choisi parmi oxygène, soufre ou azote,
- R² et R³, identiques ou différents, représentent un atome d'hydrogène ou un groupement alkyle (C₁-C₆) linéaire ou ramifié, alkoxy (C₁-C₆) linéaire ou ramifié ou hydroxyle,
   ou R² et R³ forment ensemble un groupement oxo,
- R⁴ et R⁵ représentent un atome d'hydrogène ou forment ensemble avec deux atomes adjacents du cycle B qui les portent un groupement choisi parmi aryle ou hétéroaryle,
- la notation ----- signifie que la liaison est simple ou double, étant entendu que la valence des atomes est respectée,
étant entendu que :
- le terme « substitué » affecté aux expressions « alkyle », « alkényle », « alkynyle », « cycloalkyle », « morpholinyle », « pipéridinyle », « pipérazinyle » et « pyrrolidinyle » signifie que ces groupements peuvent être substitués par un ou plusieurs groupements choisis parmi hydroxyle, oxo, alkoxy, alkyle, polyhalogénoalkyle, ou atomes d'halogène,
- le terme « substitué » affecté à l'expression « cycloalkylalkyle » signifie que la partie cyclique du groupement est substituée par un ou plusieurs groupements choisis parmi hydroxyle, oxo, alkoxy, alkyle, polyhalogénoalkyle, ou atomes d'halogène,
- par «aryle» on entend un groupement phényle, naphtyle ou biphényle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, alkoxycarbonyle, amido, ou atomes d'halogène,
- par « hétéroaryle » on entend un groupement furyle, pyrrolyle, imidazolyle, pyridyle, thiényle, pyrazinyle, benzothiényle, benzofurannyle, indolyle, benzimidazolyle, quinolyle ou quinazolinyle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, amido, ou atomes d'halogène,
leurs énantiomères et diastéréoisomères, ainsi que leurs sels d'addition à un acide ou à une base pharmaceutiquement acceptable.

Une variante avantageuse de la présente invention concerne les composés de formule (I) : 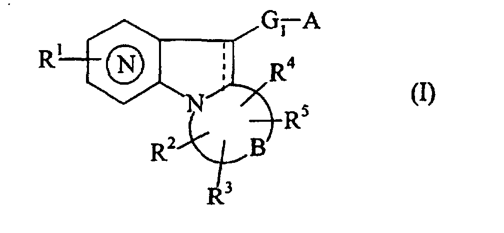 dans laquelle :
- la notation 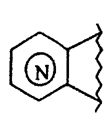 représente les groupements 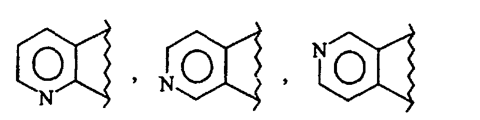 ou 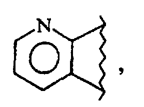
- R¹ représente un atome d'halogène ou un groupement -R, -OR, -S(O)ₙR, -NRR', 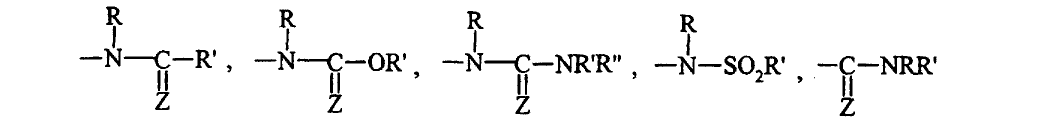 ou -SO₂NRR' (où n vaut 0, 1 ou 2, Z représente un atome de soufre ou d'oxygène, et R, R' et R", identiques ou différents, représentent un atome d'hydrogène ou un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, cycloalkyle (C₃-C₈) substitué ou non, cycloalkyl (C₃-C₈) alkyle (C₁-C₆) linéaire ou ramifié substitué ou non, aryle, arylalkyle (C₁-C₆) linéaire ou ramifié, hétéroaryle ou hétéroarylalkyle (C₁-C₆) linéaire ou ramifié,
   (R et R') et (R' et R") pouvant de plus former ensemble avec l'atome d'azote qui les porte un groupement morpholinyle, piperidinyle, piperazinyle ou pyrrolidinyle, ces groupements étant non substitués ou substitués),
- G₁ représente une chaîne alkylène comportant de 1 à 4 atomes de carbone éventuellement substituée par un groupement R, OR, COR ou COOR (où R est tel que défini précédemment),
- A représente un groupement 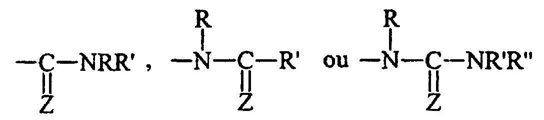 dans lesquels R, R', R" et Z sont tels que définis précédemment,
- B forme avec l'atome d'azote et l'atome de carbone auxquels il est rattaché un cycle comportant de 5 à 8 chaînons pouvant contenir une ou plusieurs insaturations et pouvant contenir, en plus de l'atome d'azote, un hétéroatome supplémentaire choisi parmi oxygène, soufre ou azote,
- R² et R³, identiques ou différents, représentent un atome d'hydrogène ou un groupement alkyle (C₁-C₆) linéaire ou ramifié, alkoxy (C₁-C₆) linéaire ou ramifié ou hydroxyle,
   ou R² et R³ forment ensemble un groupement oxo,
- R⁴ et R⁵ représentent un atome d'hydrogène ou forment ensemble avec deux atomes adjacents du cycle B qui les portent un groupement choisi parmi aryle ou hétéroaryle,
- la notation ---- signifie que la liaison est simple ou double, étant entendu que la valence des atomes est respectée,
étant entendu que :
- le terme « substitué » affecté aux expressions « alkyle », « alkényle », « alkynyle », « cycloalkyle », « morpholinyle », « pipéridinyle », « pipérazinyle » et « pyrrolidinyle » signifie que ces groupements peuvent être substitués par un ou plusieurs groupements choisis parmi hydroxyle, oxo, alkoxy, alkyle, polyhalogénoalkyle, ou atomes d'halogène,
- le terme « substitué » affecté à l'expression « cycloalkylalkyle » signifie que la partie cyclique du groupement est substituée par un ou plusieurs groupements choisis parmi hydroxyle, oxo, alkoxy, alkyle, polyhalogénoalkyle, ou atomes d'halogène,
- par « aryle » on entend un groupement phényle, naphtyle ou biphényle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, alkoxycarbonyle, amido, ou atomes d'halogène,
- par « hétéroaryle » on entend un groupement furyle, pyrrolyle, imidazolyle, pyridyle, thiényle, pyrazinyle, benzothiényle, benzofurannyle, indolyle, benzimidazolyle, quinolyle ou quinazolinyle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, amido, ou atomes d'halogène,
leurs énantiomères et diastéréoisomères, ainsi que leurs sels d'addition à un acide ou à une base pharmaceutiquement acceptable.

Une autre variante avantageuse de la présente invention concerne les composés de formule (I) : 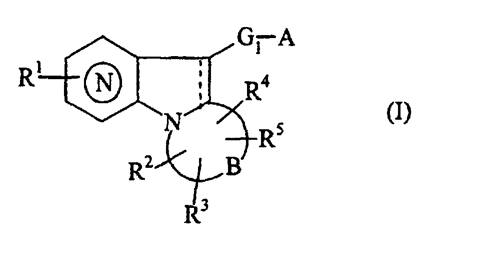 dans laquelle :
- la notation 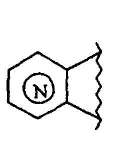 représente les groupements 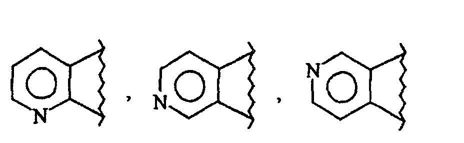 ou 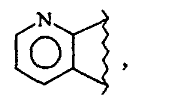
- R¹ représente un atome d'halogène ou un groupement -R, -OR, -S(O)ₙR, -NRR', 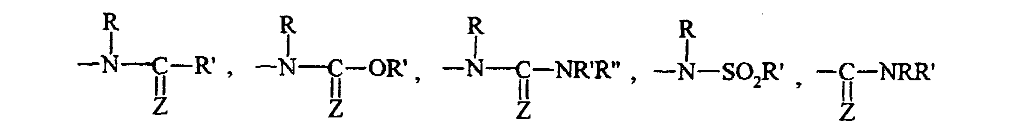 ou -SO₂NRR' (où n vaut 0, 1 ou 2, Z représente un atome de soufre ou d'oxygène, et R, R' et R", identiques ou différents, représentent un atome d'hydrogène ou un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, cycloalkyle (C₃-C₈) substitué ou non, cycloalkyl (C₃-C₈) alkyle (C₁-C₆) linéaire ou ramifié substitué ou non, aryle, arylalkyle (C₁-C₆) linéaire ou ramifié, hétéroaryle ou hétéroarylalkyle (C₁-C₆) linéaire ou ramifié,
   (R et R') et (R' et R") pouvant de plus former ensemble avec l'atome d'azote qui les porte un groupement morpholinyle, piperidinyle, piperazinyle ou pyrrolidinyle, ces groupements étant non substitués ou substitués),
- G₁ représente une chaîne alkylène comportant de 1 à 4 atomes de carbone dans laquelle un des groupements CH₂ peut être remplacé par un groupement cycloalkylène (C₃-C₈),
- A représente un groupement 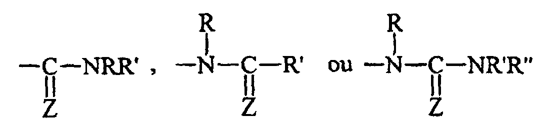 dans lesquels R, R', R" et Z sont tels que définis précédemment,
- B forme avec l'atome d'azote et l'atome de carbone auxquels il est rattaché un cycle comportant de 5 à 8 chaînons pouvant contenir une ou plusieurs insaturations et pouvant contenir, en plus de l'atome d'azote, un hétéroatome supplémentaire choisi parmi oxygène, soufre ou azote,
- R² et R³, identiques ou différents, représentent un atome d'hydrogène ou un groupement alkyle (C₁-C₆) linéaire ou ramifié, alkoxy (C₁-C₆) linéaire ou ramifié ou hydroxyle,
   ou R² et R³ forment ensemble un groupement oxo,
- R⁴ et R⁵ représentent un atome d'hydrogène ou forment ensemble avec deux atomes adjacents du cycle B qui les portent un groupement choisi parmi aryle ou hétéroaryle,
- la notation ----- signifie que la liaison est simple ou double, étant entendu que la valence des atomes est respectée,
étant entendu que :
- le terme « substitué » affecté aux expressions « alkyle », « alkényle », « alkynyle », « cycloalkyle », », morpholinyle », « pipéridinyle », « pipérazinyle » et « pyrrolidinyle » signifie que ces groupements peuvent être substitués par un ou plusieurs groupements choisis parmi hydroxyle, oxo, alkoxy, alkyle, polyhalogénoalkyle, ou atomes d'halogène,
- le terme « substitué » affecté à l'expression « cycloalkylalkyle » signifie que la partie cyclique du groupement est substituée par un ou plusieurs groupements choisis parmi hydroxyle, oxo, alkoxy, alkyle, polyhalogénoalkyle, ou atomes d'halogène,
- par « aryle » on entend un groupement phényle, naphtyle ou biphényle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, alkoxycarbonyle, amido, ou atomes d'halogène,
- par « hétéroaryle » on entend un groupement furyle, pyrrolyle, imidazolyle, pyridyle, thiényle, pyrazinyle, benzothiényle, benzofurannyle, indolyle, benzimidazolyle, quinolyle ou quinazolinyle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, amido, ou atomes d'halogène,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la *tert*butylamine, etc...

Les composés préférés de l'invention sont les composés de formule (I) pour lesquels B forme avec l'atome d'azote et l'atome de carbone auxquels il est rattaché un cycle pyrrolidine, pipéridine, (perhydro)azépine (7 chaînons), (perhydro)azocine (8 chaînons) ou un cycle contenant en plus de l'atome d'azote un atome d'oxygène et plus préférentiellement un cycle 1,3-(perhydro)oxazine.

Les groupements R² et R³ préférés des composés de formule (I) sont l'atome d'hydrogène.

Les groupements R⁴ et R⁵ préférés des composés de formule (I) sont l'atome d'hydrogène ou lorsque R⁴ et R⁵, portés par deux atomes adjacents du cycle B forment avec ces deux atomes un groupement phényle substitué ou non.

L'invention concerne plus particulièrement les composés de formule (I) pour lesquels R¹ représente un groupement OR et plus préférentiellement un groupement OR dans lequel R représente un atome d'hydrogène ou un groupement alkyle (C₁-C₆) linéaire ou ramifié comme par exemple méthyle, éthyle, propyle, butyle, pentyle, hexyle, isopropyle ou *tert*butyle.

Le groupement G₁ préféré des composés de formule (I) est le groupement (CH₂)ₚ où p vaut 2 ou 3 et plus préférentiellement 2.

Avantageusement, l'invention concerne les composés de formule (I) pour lesquels A représente un groupement NHCOR ou CONHR et plus particulièrement les groupements NHCOR ou CONHR pour lesquels R représente un groupement alkyle (C₁-C₆) linéaire ou ramifié comme par exemple méthyle, éthyle, propyle, butyle, pentyle, hexyle, isopropyle ou *tert*butyle, un groupement cycloalkyle (C₃-C₈) comme par exemple cyclopropyle, cyclobutyle, cyclopentyle ou cyclohexyle, un groupement cycloalkyl (C₃-C₈) alkyle (C₁-C₆) linéaire ou ramifié comme par exemple cyclopropylméthyle, cyclobutylméthyle, ou cyclohexylméthyle, un groupement aryle comme par exemple phényle, iodophényle, trifluorométhylphényle ou méthoxyphényle, un groupement arylalkyle (C₁-C₆) linéaire ou ramifié comme par exemple benzyle, ou un groupement hétéroaryle comme par exemple furyle, thiényle, pyrrolyle, pyridyle ou indolyle.

L'invention concerne tout particulièrement les composés de formule (I) qui sont :
* le *N*-[2-(2-méthoxy-6*H*-pyrido[2',3':4,5]pyrrolo[2,1-*a*]isoindol-11-yl)éthyl]acétamide,
* le *N*-[2-(2-méthoxy-6*H*-pyrido[2',3':4,5]pyrrolo[2,1-*a*]isoindol-11-yl)éthyl]-2-furamide,
* le *N*-[2-(2-méthoxy-6*H*-pyrido[2',3':4,5]pyrrolo[2,1-*a*]isoindol-11-yl)éthyl]benzamide,
* le *N*-[2-(3-méthoxy-6,7,8,9-tetrahydropyrido[3,2-*b*]indolizin-5-yl)éthyl]acétamide,
* le *N*-[2-(2-méthoxy-6,7,8,9-tetrahydropyrido[2,3-*b*]indolizin-10-yl)éthyl]-2-furamide,
* le *N*-[2-(2-méthoxy-6,7,8,9-tetrahydropyrido[2,3-*b*]indolizin-10-yl)éthyl]acétamide,
* le *N*-[2-(8-méthoxy-3,4-dihydro-2*H*-pyrido[2',3':4,5]pyrrolo[2,1-*b*][1,3]oxazin-10-yl) éthyl]acétamide,
* le *N*-[2-(8-méthoxy-3,4-dihydro-2*H*-pyrido[2',3':4,5]pyrrolo[2,1-*b*][1,3]oxazin-10-yl) éthyl]-2-furamide,
* le *N*-[2-(10-méthoxy-5,6-dihydropyrido[2',3':4,5]pyrrolo[2,1-*a*]isoquinolin-12-yl) éthyl]-2-furamide,
* le *N*-[2-(11-méthoxy-6,7-dihydro-5*H*-pyrido[2',3':4,5]pyrrolo[2,1-*a*][2]benzazepin-13-yl)éthyl]acétamide,
* le *N*-[2-(11-méthoxy-6,7-dihydro-5*H*-pyrido[2',3':4,5]pyrrolo[2,1-*a*][2]benzazepin-13-yl)éthyl]-2-furamide,
* le *N*-[2-(11-hydroxy-6,7-dihydro-5*H*-pyrido[2',3':4,5]pyrrolo[2,1-*a*][2]benzazepin-13-yl)éthyl]-2-furamide.

Les énantiomères, diastéréoisomères ainsi que les sels d'addition à un acide ou une base pharmaceutiquement acceptable des composés préférés de l'invention font partie intégrante de l'invention.

L'invention s'étend également au procédé de préparation des composés de formule (I) caractérisé en ce que l'on utilise comme produit de départ le composé de formule (II) : 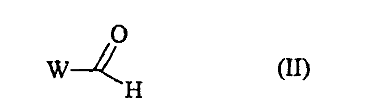 dans laquelle W représente un radical de formule (III) : 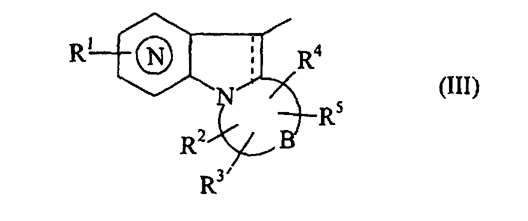 dans laquelle R¹, R², R³, R⁴, R⁵, B, la représentation 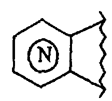 et la représentation ---- sont tels que définis précédemment, que l'on soumet
- à un agent réducteur afin d'obtenir le composé de formule (IV) :

   W-CH₂OH (IV)

   dans laquelle W est tel que défini précédemment,
   que l'on soumet après transformation en dérivé halogéné correspondant à l'action d'un sel de cyanure pour conduire au composé de formule (V) :

   W-CH₂-CN (V)

   dans laquelle W est tel que défini précédemment,
- ou successivement à une réaction de Wittig puis à une réduction pour obtenir le composé de formule (VI) :

   W-G'₁-X (VI)

   dans laquelle W est tel que défini précédemment , X représente un groupement CN ou COOalk (où alk représente un groupement alkyle) et G'₁ représente une chaîne telle que définie précédemment pour G₁ comportant 2 à 4 atomes de carbones,
composés de formules (V) et (VI) que l'on hydrolyse en milieu acide ou basique pour obtenir le composé de formule (VII) :

W-G₁-COOH (VII)

dans laquelle W et G₁ sont définis comme précédemment,
sur lequel on fait agir, en présence d'un agent de couplage, ou après transformation en chlorure d'acide correspondant, une amine HNRR' où R et R' sont définis comme précédemment pour conduire au composé de formule (I/a), cas particulier des composés de formule (I) : 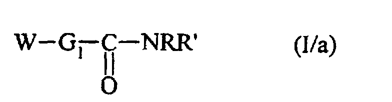 dans laquelle W, G₁, R et R' sont définis comme précédemment,
composé de formule (I/a) qui est soumis, dans le cas où R et R' représentent simultanément un atome d'hydrogène, à l'action de NaOBr afin de conduire, après hydrolyse, au composé de formule (VIII) :

W-G₁-NH₂ (VIII)

dans laquelle W et G₁ sont définis comme précédemment,
que l'on soumet à l'action :
- d'un chlorure d'acyle de formule (IX) : 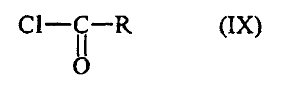 où R est tel que défini précédemment, ou l'anhydride d'acide (mixte ou symétrique) correspondant,
   pour obtenir le composé de formule (I/b), cas particulier des composés de formule (I) : 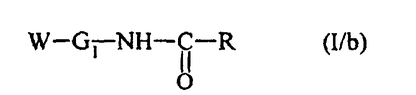 dans laquelle W, G₁ et R sont définis comme précédemment,
   composé de formule (I/b) pouvant par ailleurs être obtenu lorsque G₁ représente une chaîne (CH₂)₂, à partir du composé de formule (II) par action du nitrométhane pour conduire au composé de formule (III') : 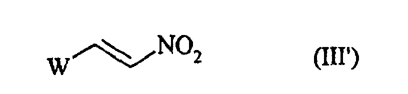 dans laquelle W est défini comme précédemment,
   qui est soumis à l'action d'un agent réducteur comme NaBH₄, par exemple, pour obtenir le composé de formule (IV') : 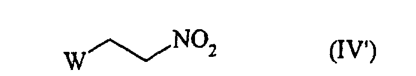 dans laquelle W est tel que défini précédemment,
   que l'on soumet à une hydrogénation catalytique afin d'obtenir le composé de formule (V') : 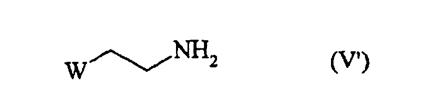 dans laquelle W est tel que défini précédemment,
   sur lequel on fait agir un chlorure d'acide de formule (IX) ou l'anhydride d'acide (mixte ou symétrique) correspondant pour obtenir le composé de formule (I/b'), cas particulier des composés de formule (I/b) : 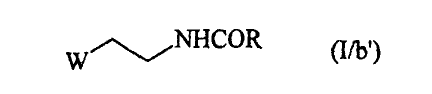 dans laquelle W et R sont définis comme précédemment,
- ou composé de formule (VIII) que l'on soumet à l'action d'un composé de formule (X) :

   O=C=N-R (X)

   dans laquelle R est tel que défini précédemment,
   pour conduire au composé de formule (I/c), cas particulier des composés de formule (I) : 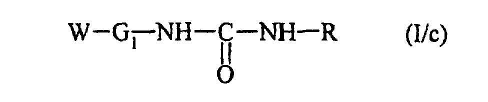 dans laquelle W, G₁ et R ont la même définition que précédemment,
les composés de formule (I/b) et (I/c) pouvant être soumis à l'action d'un composé de formule (XI) :

Rₐ-J (XI)

dans laquelle Rₐ peut prendre toutes les valeurs de R à l'exception de l'atome d'hydrogène et J représente un groupe partant comme un atome d'halogène ou un groupement tosyle,
pour conduire au composé de formule (I/d), cas particulier des composés de formule (I) : 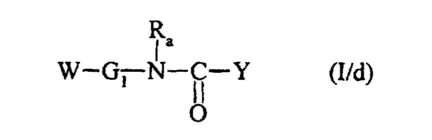 dans laquelle W, G₁ et Rₐ sont tels que définis précédemment et Y représente un groupement R ou -NRR' où R et R' sont tels que définis précédemment,
et/ou les composés de formules (I/a), (I/b), (I/c) et (I/d) peuvent être soumis à l'action d'un agent de thionation, comme le réactif de Lawesson pour conduire au composé de formule (I/e), cas particulier des composés de formule (I) :

W-G₁-T (I/e)

dans laquelle W et G₁ sont définis de la même façon que précédemment et T représente un groupement 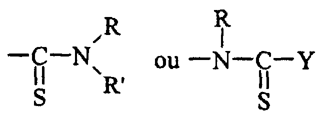 dans lesquels R, R' et Y sont tels que définis précédemment,
les composés (I/a) à (I/e) formant l'ensemble des composés de formule (I) et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

Les composés de formule (III) sont, soit commerciaux, soit accessibles à l'homme du métier par des réactions chimiques classiques.

En particulier, les composés de formule (III) peuvent être obtenus à partir du composé de formule (XII) : 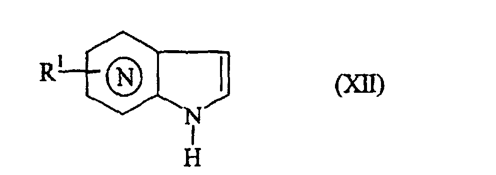 dans laquelle R¹ et la représentation 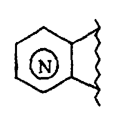 sont définis comme précédemment, qui est soit commercial, soit obtenu selon un mode opératoire décrit par G. Guillaumet et col. (Heterocycles, 1999, 50 (2), pp. 1065-1079),
sur lequel on condense un composé de formule (XIII) :

Hal-G₂-Hal (XIII)

dans laquelle Hal représente un atome d'halogène et G₂ représente une chaîne contenant 3 à 6 chaînons substituée par les groupements R², R³, R⁴ et R⁵ tels que définis précédemment, pouvant contenir une ou plusieurs insaturations et contenant éventuellement un hétéroatome choisi parmi oxygène, azote et soufre,
pour conduire au composé de formule (XIV) : 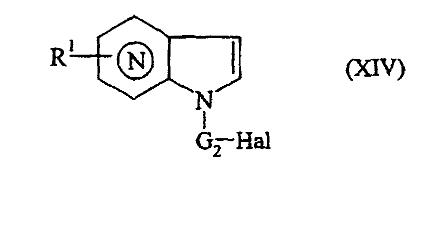 dans laquelle R¹, G₂, Hal et la représentation 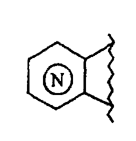 ont la même définition que précédemment,
- que l'on transforme, par action successive de brome dans tBuOH puis de Zinc dans l'acide acétique en composé de formule (XV) : 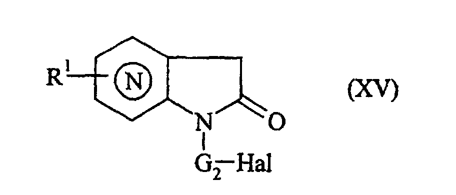 dans laquelle R¹, G₂, Hal et la représentation 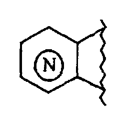 sont tels que définis précédemment,
- ou que l'on formyle par action de POCl₃ dans le diméthylformamide (DMF) pour conduire au composé de formule (XVI) : 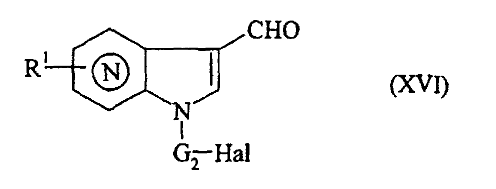 dans laquelle R¹, G₂, Hal et la représentation 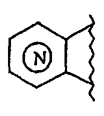 sont tels que définis précédemment, (le composé de formule (XVI) pouvant également être obtenu à partir du composé de formule (XII) que l'on soumet successivement à une formylation puis à la condensation du composé de formule (XIII)),
   composé de formule (XVI) que l'on place dans des conditions de cyclisation comme Bu₃SnH/AIBN pour conduire au composé de formule (III),
   les composés de formules (XIV) et (XV) pouvant être également soumis à des conditions de cyclisation en utilisant, selon le cas des réactifs comme NaH dans le DMF ou (PPh₃)₄ Pd par exemple, pour conduire au composé de formule (XVII) : 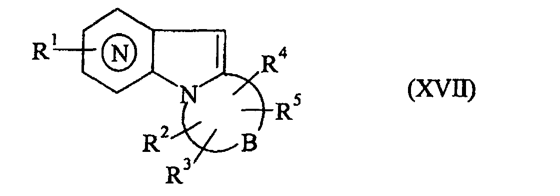 dans laquelle R¹, R², R³, R⁴, R⁵, B et la représentation 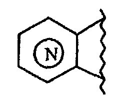 sont tels que définis précédemment,
   que l'on soumet à des conditions de formylation comme POCl₃ dans le DMF pour obtenir le composé de formule (III).

La présente invention concerne également les composés de formule (XVII') : 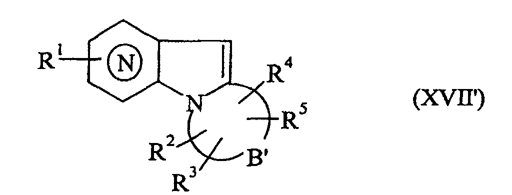 dans laquelle R¹, R², R³, R⁴, R⁵ et la représentation 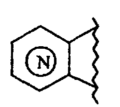 sont tels que définis dans la formule (I) et B' représente un groupement B tel que défini dans la formule 1 à l'exception du groupement pipérazinyle utiles en tant qu'intermédiaires de synthèse de formule (I).

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une très haute affinité pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation, qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon. Les préparations suivantes conduisent à des intermédiaires de synthèse utiles dans la préparation des composés de l'invention.

### Préparation 1: 2-(2-Méthoxy-6H-pyrido[2',3':4,5]pyrrolo[2,1-a]isoindol-11-yl) éthylamine

### Stade A : 2-(6-Méthoxy-3 -nitro -2-pyridinyl)acétonitrile

Sous argon et en milieu anhydre, 15 g (97,3 mmol) de 2-méthoxy-5-nitropyridine et 17,9 g (107 mmol) de 4-chlorophénoxyacétonitrile sont dissous dans 300 ml de tétrahydrofurane anhydre ; la solution est additionnée par transfert à une solution de 24 g (214 mmol) de *tert*-butylate de potassium dans 220 ml de tétrahydrofurane anhydre maintenue à une température inférieure à -10°C. La réaction est agitée pendant trois heures à une température comprise entre -10 et -15°C. Une solution aqueuse d'acide chlorhydrique à 5 % (170 ml) est ajoutée goutte à goutte à la réaction en maintenant la température à -10°C. Le milieu est extrait par l'acétate d'éthyle. Après séchage sur sulfate de magnésium et évaporation, le résidu est purifié par chromatographie flash sur gel de silice (éluant : éther de pétrole/acétate d'éthyle, 2 : 1). Après lavage par un mélange éther de pétrole/éther diéthylique (2:1), le composé du titre est obtenu sous la forme d'un produit brun.
Point de fusion : 116-117°C

### Stade B : 5-Méthoxy-1H-pyrrolo[3,2-b]pyridine

Sous une pression de 45 psi d'hydrogène, 17,70 g (91,7 mmol) du composé obtenu au stade A et 2,5 g de palladium sur charbon en suspension dans 300 ml d'éthanol sont agités pendant 5 heures à température ambiante. Après filtration sur célite et évaporation, le résidu est purifié sur gel de silice (éluant : éther de pétrole/acétate d'éthyle, 4 : 1) pour conduire au produit du titre sous la forme d'un solide brun.
Point de fusion : 111-112°C

### Stade C : 1-(2-Bromobenzyl)-5-méthoxy-1H-pyrrolo[3,2-b]pyridine

Sous argon et en milieu anhydre, 590 mg (14,75 mmol) d'hydrure de sodium (60 % dans l'huile) sont additionnés lentement à une solution de 1,81 g (12,23 mmol) du composé obtenu au stade B dans 50 ml de *N,N*-diméthylformamide à 0°C. Après 30 minutes d'agitation à température ambiante, 2,27 ml (14,75 mmol) de bromure de 2-bromobenzyle dans 15 ml de *N,N*-diméthylformamide sont additionnés goutte à goutte au milieu réactionnel. L'agitation est maintenue à température ambiante sous argon pendant deux heures. Après hydrolyse puis extraction par l'acétate d'éthyle, la phase organique est lavée plusieurs fois à l'eau, séchée sur sulfate de magnésium, concentrée et purifiée par chromatographie flash sur gel de silice (éluant : éther de pétrole/acétate d'éthyle, 8 : 2) pour conduire au composé du titre sous la forme d'un solide brun.
Point de fusion : 86-87°C

### Stade D : 2-Méthoxy-6H-pyrido[2',3':4,5]pyrrolo[2,1-a]isoindole

Sous atmosphère inerte, 500 mg (1,57 mmol) du composé obtenu au stade C, 88 mg (78,9 µmol) de tétrakis(triphénylphosphine)palladium(0) et 155 mg (1,57 mmol) d'acétate de potassium dans 15 ml de *N,N*-diméthylacétamide sont chauffés à 160°C pendant 2 heures. Après retour à température ambiante, le milieu réactionnel est filtré sur büchner puis évaporé à sec. Le résidu est ensuite hydrolysé puis extrait par l'acétate d'éthyle. La phase organique est séchée sur sulfate de magnésium, concentrée et purifiée par chromatographie flash sur gel de silice (éluant : éther de pétrole/acétate d'éthyle, 8 : 2) pour conduire au composé du titre sous la forme d'un solide brun.
Point de fusion : 192-193°C

### Stade E : 2-Méthoxy-6H-pyrido[2',3':4,5]pyrrolo[2,1-a]isoindole-11-carbaldéhyde

Sous argon et en milieu anhydre, 291 µl (3,17 mmol) d'oxychlorure de phosphore sont ajoutés goutte à goutte à 6 ml de *N,N*-diméthylformamide maintenus à 0°C ; après 15 minutes d'agitation, 500 mg (2,11 mmol) du composé obtenu au stade D en solution dans 10 ml de *N,N*-diméthylformamide sont additionnés par transfert. La réaction est agitée 30 minutes à 0°C puis 2 heures à température ambiante. Le mélange réactionnel est évaporé à sec puis repris dans l'eau ; la phase aqueuse est alcalinisée par une solution de soude 6 N et extraite plusieurs fois par l'acétate d'éthyle. Les phases organiques réunies sont séchées sur sulfate de magnésium, filtrées et évaporées. Après lavage par un mélange éther de pétrole/éther diéthylique, le composé du titre est obtenu sous la forme d'un solide brun.
Point de fusion : 229-230°C

### Stade F : 2-Méthoxy-11-[2-nitroéthényl]-6H-pyrido[2',3':4,5]pyrrolo[2,1-a] isoindole

600 mg (2,27 mmol) du composé obtenu au stade E dissous dans 25 ml de nitrométhane sont chauffés à 120°C pendant 3 heures en présence de 440 mg (5,67 mmol) d'acétate d'ammonium. Après retour à température ambiante et évaporation à sec, la réaction est diluée dans le dichlorométhane et lavée avec de l'eau. Après séchage sur sulfate de magnésium, l'évaporation de la phase organique conduit au composé du titre sous la forme d'un solide jaune.
Point de fusion : 197-198°C

### Stade G : 2 -Méthoxy-11-(2-nitroéthyl)-6H-pyrido[2',3':4,5]pyrrolo[2,1-a] isoindole

Sous argon et en milieu anhydre, 660 mg (2,15 mmol) du dérivé obtenu au stade F sont mis en suspension dans 10 ml d'isopropanol et 30 ml de chloroforme en présence de 1,5 g de silice 230-400 mesh ; 408 mg (10,75 mmol) de borohydrure de sodium sont lentement ajoutés. Après 30 minutes d'agitation à température ambiante, de l'acide acétique est ajouté et la réaction est filtrée sur verre fritté. Après évaporation des solvants, le résidu est dilué dans le dichlorométhane et lavé avec de l'eau. La phase organique est ensuite séchée sur sulfate de magnésium, filtrée et concentrée pour conduire au composé du titre sous la forme d'un solide jaune.
Point de fusion : 127-128°C

### Stade H : 2-(2-Méthoxy-6H-pyrido[2',3':4,5]pyrrolo[2,1-a]isoindol-11-yl) éthylamine

Sous une pression de 55 psi d'hydrogène, 530 mg (1,71 mmol) du composé obtenu au stade G et 210 mg de nickel de Raney sont agités dans 20 ml de méthanol à 60°C pendant 15 heures. Après retour à température ambiante, la réaction est filtrée sur büchner puis évaporée pour conduire à l'amine du titre sous la forme d'une huile brune.
Point de fusion (oxalate) : 149-150°C

### Préparation 2: 2-[2-Méthoxy-8-(triflurométhyl)-6H-pyrido[2'3':4,5]pyrrolo[2,1-α] isoindol-11-yl]éthylamine

On procède comme dans la Préparation 1 en remplaçant au stade C le bromure de 2-bromobenzyle par le bromure de (4-trifluorométhyl)-2-bromobenzyle.

### Préparation 3 : Acide 4-(2-méthoxy-6H-pyrido[2',3':4,5]pyrrolo[2,1-a]isoindol-11-yl] butanoïque

### Stade A : 4-(2-Méthoxy-6H-pyrido[2',3': 4,5]pyrrolo[2,1-a]isoindol-11-yl)-3-buténoate d'éthyle

10,7 mmol du composé obtenu au stade E de la Préparation 1 dans 20 ml de THF anhydre sont mis en présence de 1,2 éq de NaH (60 % dans l'huile) dans 25 ml de THF anhydre et de 1,2 éq de 3-(diéthoxyphosphoryl)propanoate d'éthyle. Après 3 heures d'agitation à température ambiante et une nuit au léger reflux, le composé du titre est obtenu.

### Stade B : Acide 4-(2-méthoxy-6H-pyrido[2',3':4,5]pyrrolo[2,1-a]isoindol-11-yl) butanoique

L'ester obtenu au stade A est placé dans 150 ml d'éthanol en présence de 40 ml de soude 2 N. Après agitation à l'ambiante, puis au reflux, l'acide du titre est isolé.

### Préparation 4: 2-(10-Méthoxy-5,6-dihydropyrido[2',3':4,5]pyrrolo[2,1-a] isoquinolin-12-yl)éthylamine

### Stade A : 1-{[2- (2-Bromophényl)-1,3-dioxolan-2-yl]méthyl}-5-méthoxy-1H-pyrrolo[3,2-b]pyridine

Sous argon et en milieu anhydre, 295 mg (2,6 mmol) de *tert*-butylate de potassium sont ajoutés à une solution contenant 300 mg (2 mmol) du composé obtenu au stade B de la Préparation 1 et 785 mg (2,4 mmol) de 2-(bromoéthyl)-2-(2-bromophényl)-1,3-dioxolane dans 5 ml de diméthylsulfoxide. Le mélange réactionnel est chauffé à 100°C pendant 24 heures. Après retour à température ambiante, le milieu est hydrolysé par de l'eau puis extrait par l'acétate d'éthyle. Après plusieurs lavages à l'eau, la phase organique est séchée sur sulfate de magnésium, filtrée puis évaporée. Une purification par chromatographie flash sur gel de silice (éluant : éther de pétrole/acétate d'éthyle, 85: 15) conduit au composé du titre sous la forme d'un solide blanc.
Point de fusion : 143-144°C

### Stade B :1-(2-Bromophényl)-2-(5-méthoxy-1H-pyrrolo[3,2-b]pyridin-1-yl)-1-éthanone

Une solution contenant 1,60 g (4,1 mmol) du composé obtenu au stade A dans 10 ml de méthanol et 20 ml d'acide chlorhydrique à 18 % est portée à reflux pendant 4 heures. Après retour à température ambiante et évaporation du méthanol, le milieu est neutralisé par une solution saturée d'hydrogénocarbonate de sodium puis extrait par l'acétate d'éthyle. La phase organique est séchée sur sulfate de magnésium, filtrée et concentrée. Le résidu est purifié par chromatographie flash sur gel de silice (éluant: éther de pétrole/acétate d'éthyle, 8 : 2) pour conduire au composé du titre sous forme d'un solide blanc.
Point de fusion : 116-117°C

### Stade C : 1-(2-Bromophénéthyl)-5-méthoxy-1H-pyrrolo[3,2-b]pyridine

140 µl (2,9 mmol) d'hydrate d'hydrazine et 80 mg (2 mmol) de soude sont ajoutés à une solution de 200 mg (0,6 mmol) du composé obtenu au stade B dans 2 ml de diéthylèneglycol. Après 24 heures d'agitation à 160°C, le milieu est hydrolysé puis extrait par l'acétate d'éthyle. La phase organique est lavée plusieurs fois à l'eau, séchée sur sulfate de magnésium, concentrée puis purifiée par chromatographie flash sur gel de silice (éluant : éther de pétrole/acétate d'éthyle, 7 : 3) pour conduire au composé du titre sous la forme d'une huile jaune.

### Stade D : 10-Méthoxy-5,6-dihydropyrido[2',3':4,5]pyrrolo[2,1-a]isoquinoline

Le composé du titre est obtenu à partir du composé obtenu au stade C selon le protocole expérimental décrit au stade D de la Préparation 1. Solide jaune.
Point de fusion : 179-180°C

### Stade E : 10-Méthoxy-5,6-dihydropyrido[2',3':4,5]pyrrolo[2,1-a]isoquinoline-12-carbaldéhyde

On utilise le même procédé qu'au stade E de la Préparation 1. Solide jaune.
Point de fusion : 192-193°C

### Stade F: 2-(10-Méthoxy-5,6-dihydropyrido[2',3':4,5]pyrrolo[2,1-a] isoquinolin-12-yl)éthylamine

On procède comme aux stades F, G et H de la Préparation 1. Huile brune.
Point de fusion (oxalate) : 137-138°C

### Préparation 5 : 2-(11-Méthoxy-6,7-dihydro-5H-pyrido[2',3':4,5]pyrrolo[2,1-a][2] benzazépin-13-yl)éthylamine

### Stade A : 1-[3-(2-Bromophényl)propyl]-5-méthoxy-1H-pyrrolo[3,2-b]pyridine

Sous argon et en milieu anhydre, 162 mg (4,05 mmol) d'hydrure de sodium (60 % dans l'huile) sont lentement additionnés, à 0°C, à une solution de 500 mg (3,38 mmol) du composé obtenu au stade B de la Préparation 1 dans 10 ml de *N,N*-diméthylformamide. Après 35 minutes d'agitation à température ambiante, une solution de 1,3 g (4,05 mmol) de 1-bromo-2-(3-iodopropyl)-benzène en solution dans 5 ml de *N,N*-diméthylformamide sont additionnés au mélange. Après 1 heure d'agitation à température ambiante, le milieu est hydrolysé puis extrait par l'acétate d'éthyle. La phase organique est lavée plusieurs fois à l'eau, séchée sur sulfate de magnésium, concentrée et purifiée par chromatographie flash sur gel de silice (éluant : éther de pétrole/acétate d'éthyle, 8 : 2) pour conduire au composé du titre sous la forme d'une huile jaune.

### Stade B: 11-Méthoxy-6,7-dihydro-5H-pyrido[2',3':4,5]pyrrolo[2,1-a][2] benzazépine

Sous atmosphère inerte, 500 mg (1,45 mmol) du composé obtenu au stade A, 81 mg (72,5 µmol) de tétrakis(triphénylphosphine) palladium(0) et 142 mg (1,45 mmol) d'acétate de potassium dans 15 ml de *N,N*-diméthylacétamide sont chauffés à 160°C pendant 2 heures. Après retour à température ambiante, le milieu réactionnel est filtré sur büchner puis évaporé à sec. Le résidu est ensuite hydrolysé puis extrait par l'acétate d'éthyle. La phase organique est séchée sur sulfate de magnésium, concentrée et purifiée par chromatographie flash sur gel de silice (éluant : éther de pétrole/acétate d'éthyle, 9 : 1) pour conduire au composé du titre sous la forme d'une huile incolore.

### Stade C : 11-Méthoxy-6,7-dihydro-5H-pyrido[2',3':4,5]pyrrolo[2,1-a][2] benzazépin-13-carbaldéhyde

On procède comme dans le stade F de la Préparation 1.

### Stade D : 2-(11-Méthoxy-6,7-dihydro-5H-pyrido[2',3':4,5]pyrrolo[2,1-a][2] benzazépin-13-yl)éthylamine

On procède comme dans les stades G et H de la Préparation 1. Solide vert.
Point de fusion : 114-115°C

### Préparation 6: Acide 4-(6,7-dihydro-5H-pyrido[2',3':4,5]pyrrolo[2,1-a][2] benzazépin-13-yl)butanoïque

On procède comme dans la Préparation 3 à partir du 6,7-dihydro-5*H*-pyrido[2',3':4,5]pyrrolo[2,1-*a*][2]benzazépin-13-carbaldéhyde (obtenu selon un procédé analogue aux stades A, B et C de la Préparation 5).

### Préparation 7: 2-(11-Ethyl-6,7-dihydro-5H-pyrdo[2',3':4,5]pyrrolo[2,1-a][2] benzazépin-13-yl)éthylamine

On procède comme dans la Préparation 5 à partir de la 2-éthyl-5-nitropyridine.

### Préparation 8: 2-(2-Méthoxy-6,7,8,9-tétrahydropyrido[2,3-b]indolizin-10-yl)éthylamine

### Stade A : 1-(4-Bromobutyl)-5-méthoxy-1H-pyrrolo[3,2-b]pyridine

Sous argon et en milieu anhydre, 810 mg (20,27 mmol) d'hydrure de sodium (60 % dans l'huile) sont additionnés lentement à une solution de 2 g (13,51 mmol) du composé obtenu au stade B de la Préparation 1 dans 30 ml de *N,N*-diméthylformamide à 0°C. Après une heure d'agitation à température ambiante, la solution contenant l'anion préalablement formé est additionnée goutte à goutte à 4,8 ml (40,54 mmol) de 1,4-dibromobutane. Après deux heures d'agitation à température ambiante, le milieu réactionnel est hydrolysé puis extrait par l'acétate d'éthyle. La phase organique est séchée sur sulfate de magnésium, concentrée et purifiée par chromatographie flash sur gel de silice (éluant : éther de pétrole/acétate d'éthyle, 8 : 2) pour conduire au composé du titre sous la forme d'une huile incolore.

### Stade B: 1-(4-Bromobutyl)-5-méthoxy-1H-pyrrolo[3,2-b]pyridine-3-carbaldéhyde

Sous argon et en milieu anhydre, 730 µl (7,95 mmol) d'oxychlorure de phosphore sont ajoutés goutte à goutte à 12,5 ml de *N,N*-diméthylformamide maintenus à 0°C ; après 30 minutes d'agitation, 1,50 g (5,3 mmol) du composé obtenu au stade A en solution dans 25 ml de *N,N*-diméthylformamide sont additionnés par transfert. La réaction est agitée 30 minutes à 0°C puis 3 heures à température ambiante. Le mélange réactionnel est évaporé à sec puis repris dans l'eau ; la phase aqueuse est alcalinisée par une solution de soude 6 N et extraite plusieurs fois par l'acétate d'éthyle. Les phases organiques réunies sont séchées sur sulfate de magnésium, filtrées puis évaporées. Après lavage par un mélange éther de pétrole/éther diéthylique, le composé du titre est obtenu sous la forme d'une huile jaune.

### Stade C: 2-Méthoxy-6,7,8,9-tétrahydropyrido[2,3-b]indolizine-10-carbaldéhyde

Sous argon, une solution contenant 2,70 g (8,67 mmol) du composé obtenu au stade B et 1,45 g (8,67 mmol) d'AIBN dans 80 ml de toluène est portée à reflux. Après 20 minutes d'agitation, 4,67 ml (17,35 mmol) d'hydrure de tributylétain dans 100 ml de toluène sont additionnés au milieu réactionnel qui est maintenu à reflux pendant 24 heures. Après retour à température ambiante et évaporation à sec, le résidu est ensuite hydrolysé puis extrait par l'acétate d'éthyle. La phase organique est séchée sur sulfate de magnésium, concentrée et purifiée par chromatographie flash sur gel de silice (éluant : éther de pétrole/acétate d'éthyle, 95 : 5) pour conduire au produit du titre sous la forme d'une huile jaune.

### Stade D : 2-Méthoxy-10-[2-nitroéthényl]-6,7,8,9-tétrahydropyrido[2,3-b]indolizine

550 mg (2,39 mmol) du composé obtenu au stade C dissous dans 25 ml de nitrométhane sont chauffés à 120°C pendant 3 heures en présence de 460 mg (5,97 mmol) d'acétate d'ammonium. Après retour à température ambiante et évaporation à sec, la réaction est diluée dans le dichlorométhane et lavée avec de l'eau. La phase organique est séchée sur sulfate de magnésium, filtrée et évaporée. Après lavage par un mélange pentane/éther diéthylique, le composé du titre est obtenu sous la forme d'un solide brun.
Point de fusion : 202-203°C

### Stade E : 2-Méthoxy-10-(2-nitroéthyl)-6,7,8,9-tétrahydropyrido[2,3-b]indolizine

Sous argon et en milieu anhydre, 650 mg (2,38 mmol) du dérivé obtenu au stade D sont mis en suspension dans 10 ml d'isopropanol et 30 ml de chloroforme en présence de 1,5 g de silice 230-400 mesh ; 450 mg (11,90 mmol) de borohydrure de sodium sont lentement ajoutés. Après 30 minutes d'agitation à température ambiante, de l'acide acétique est ajouté et la réaction est filtrée sur verre fritté. Après évaporation des solvants, le résidu est dilué dans le dichlorométhane et lavé avec de l'eau. La phase organique est ensuite séchée sur sulfate de magnésium, filtrée et concentrée pour conduire au composé du titre sous la forme d'un solide jaune.
Point de fusion : 96-97°C

### Stade F : 2-(2-Méthoxy-6,7,8,9-tétrahydropyrido[2,3-b]indolizin-10-yl)éthylamine

Sous une pression de 55 psi d'hydrogène, 420 mg (1,53 mmol) du composé obtenu au stade E et 170 mg de nickel de Raney sont agités dans 20 ml de méthanol à 60°C pendant 15 heures. Après retour à température ambiante, la réaction est filtrée sur büchner puis évaporée pour conduire au produit du titre sous la forme d'une huile brune.

### Préparation 9: 2-(3-Méthoxy-6,7,8,9-tétrahydropyrido[3,2-b]indolizin-5-yl)éthylamine

### Stade A : 3,3,5-Tribromo-1,3-dihydro -2H-pyrrolo[2,3-b]pyridin-2-one

A température ambiante, 54 ml (1,05 mol) de dibrome sont ajoutés goutte à goutte à 10 g (84 mmol) de 7-azaindole dissous dans 660 ml de *tert*-butanol et 660 ml d'eau. Après 19 heures d'agitation à température ambiante, le *tert*-butanol est évaporé et la phase aqueuse résiduelle est alcalinisée par une solution aqueuse saturée d'hydrogénocarbonate de sodium. Le produit désiré est récupéré par filtration. Après séchage sous vide en présence de pentoxyde de phosphore, le composé du titre est obtenu sous la forme d'un solide brun.
Point de fusion : 157-158°C

### Stade B : 5-Bromo-1,3-dihydro-2H-pyrrolo[2,3-b]pyridin-2-one

Sous argon et à température ambiante, 17,60 g (0,27 mol) de zinc en poudre sont ajoutés portion par portion à 5 g (13,5 mmol) du composé obtenu au stade A dissous dans 100 ml d'acide acétique. Après 15 heures d'agitation à température ambiante, l'acide acétique est évaporé sous pression réduite (coévaporation au toluène). Le résidu, repris par l'acétate d'éthyle, est lavé une fois par l'eau ; la phase aqueuse est lavée plusieurs fois à l'acétate d'éthyle. Les phases organiques sont rassemblées, séchées sur sulfate de magnésium, concentrées et purifiées par chromatographie flash sur gel de silice (éluant : dichlorométhane/méthanol, 95/5), pour obtenir le composé du titre sous la forme d'un solide orangé.
Point de fusion : 250-251°C

### Stade C : 5-Bromo-1H-pyrrolo[2,3-b]pyridine

Sous atmosphère d'argon et en milieu anhydre, 37,6 ml (37,6 mmol) d'une solution 1 M de complexe borane-tétrahydrofurane dans le tétrahydrofurane sont additionnés à 0°C et goutte à goutte à une suspension de 2 g (9,4 mmol) du composé obtenu au stade B dans 50 ml de tétrahydrofurane anhydre. La réaction est agitée 35 minutes à température ambiante puis évaporée à sec. Le résidu est repris par une solution aqueuse d'acide chlorhydrique 6 M et chauffé jusqu'à dissolution complète du solide. Après refroidissement, la solution est alcalinisée par une solution aqueuse de soude 6 M et extraite par l'acétate d'éthyle. Après séchage sur sulfate de magnésium et évaporation, un mélange équimolaire de 5-bromo-7-azaindoline et de 5-bromo-7-azaindole est obtenu. Le résidu précédent, mis en solution dans 20 ml d'acide acétique, est ajouté à température ambiante à une suspension de 4,10 g (15,3 mmol) d'acétate de manganèse III dihydraté dans 20 ml d'acide acétique. Après 45 minutes d'agitation à 75°C, la solution est évaporée à sec et coévaporée avec du toluène. Le résidu, repris dans l'eau, est alcalinisé par une solution aqueuse saturée d'hydrogénocarbonate de sodium. Après extraction par l'acétate d'éthyle, les phases organiques sont séchées sur sulfate de magnésium et évaporées. Le résidu obtenu est purifié par chromatographie flash sur gel de silice (éluant : éther de pétrole/acétate d'éthyle) pour conduire au composé du titre sous la forme d'un solide jaune.
Point de fusion : 176-177°C

### Stade D : 5-Méthoxy-1H-pyrrolo[2,3-b]pyridine

Sous atmosphère d'argon et en milieu anhydre, 1 g (5,07 mmol) du composé obtenu au stade C est dissous dans un mélange de 25 ml de *N,N*-diméthylformamide et 22 ml de méthanol ; 6,86 g (126,75 mmol) de méthanolate de sodium et 1,43 g (10,30 mmol) de bromure cuivreux sont ajoutés à température ambiante. Le mélange est chauffé à reflux pendant 3 heures. Après évaporation des solvants, le résidu est repris dans l'eau et l'acétate d'éthyle puis filtré sur célite. La phase organique est lavée avec de l'eau, séchée sur sulfate de magnésium et évaporée pour donner un solide qui est purifié par chromatographie flash sur gel de silice (éluant : éther de pétrole/acétate d'éthyle, 7 : 3). Le composé du titre est obtenu sous la forme d'un solide brun.
Point de fusion : 162-163°C

### Stade E : 2 -(3-Méthoxy-6,7,8,9-tétrahydropyrido[3,2-b]indolizin-5-yl)éthylamine

On procède comme dans les stades A, B, C, D, E et F de la Préparation 8 à partir du composé obtenu au stade D. Huile brune.

### Préparation 10: Acide 4-(2-Méthoxy-6,7,8,9-tétrahydropyrido[2,3-b]indolizin-10-yl)butanoïque

On procède comme dans la Préparation 3 à partir du composé obtenu au stade C de la Préparation 8.

### Préparation 11: 2-(2-Méthoxy-7,8-dihydro-6H-pyrido[2,3-b]pyrrolizin-9-yl) éthylamine

On procède comme dans la Préparation 8 en remplaçant au stade A le 1,4-dibromobutane par le 1,4-dibromopropane.

### Préparation 12: 2-(2-Isopropyl-7,8-dihydro-6H-pyrido[2,3-b]pyrrolizin-9-yl) éthylamine

On procède comme dans la Préparation 8 en remplaçant au stade A le 5-méthoxy-1*H*-pyrrolo[3,2-*b*]pyridine par le 5-isopropyl-1*H*-pyrrolo[3,2-*b*]pyridine et le 1,4-dibromobutane par le 1,3-dibromopropane.

### Préparation 13: 2-[2-(Benzyloxy)-7,8,9,10-tétrahydro-6H-pyrido[2',3':4,5]pyrrolo [1,2-a]azépin-11-yl]éthylamine

On procède comme dans la Préparation 8 en remplaçant au stade A le 5-méthoxy-1*H*-pyrrolo[3,2-*b*]pyridine par le 5-benzyloxy-1*H*-pyrrolo[3,2-*b*]pyridine et le 1,4-dibromobutane par le 1,5-dibromopentane.

### Préparation 14: 2-(8-Méthoxy-3,4-dihydro-2H-pyrido[2',3':4,5]pyrrolo[2,1-b][1,3] oxazin-10-yl)éthylamine

### Stade A : 1-[3-(Benzyloxy)propyl]-5-méthoxy-1H-pyrrolo[3,2-b]pyridine

Sous argon et en milieu anhydre, 973 mg d'hydrure de sodium (60 % dans l'huile) (24,32 mmol) sont lentement additionnés, à 0°C, à une solution de 3 g (20,27 mmol) du composé obtenu au stade B de la Préparation 1 dans 90 ml de *N,N*-diméthylformamide. Après 45 minutes d'agitation à température ambiante, une solution de 4,3 ml (24,32 mmol) de 1-benzyloxy-3-bromopropane en solution dans 10 ml de *N,N*-diméthylformamide sont additionnés au mélange. Après 2 heures d'agitation à température ambiante, le milieu est hydrolysé puis extrait par l'acétate d'éthyle. La phase organique est lavée plusieurs fois à l'eau, séchée sur sulfate de magnésium, concentrée et purifiée par chromatographie flash sur gel de silice (éluant : éther de pétrole/acétate d'éthyle, 7 : 3) pour conduire au composé du titre sous la forme d'une huile jaune.

### Stade B : 1-[3-(Benzyloxy)propyl]-3,3-dibromo-5-méthoxy-1,3-dihydro-2H-pyrrolo[3,2-b]pyridin-2-one

A température ambiante, 1,74 ml (33,78 mmol) de dibrome sont ajoutés goutte à goutte à 2 g (6,75 mmol) du composé obtenu au stade A dissous dans 55 ml de *tert*-butanol et 55 ml d'eau. Après 10 heures d'agitation à température ambiante, le *tert*-butanol est évaporé et la phase aqueuse résiduelle est alcalinisée par une solution aqueuse saturée d'hydrogénocarbonate de sodium puis extraite par le dichlorométhane. La phase organique est séchée sur sulfate de magnésium, filtrée et concentrée. Le résidu est purifié par chromatographie flash sur gel de silice (éluant : éther de pétrole/acétate d'éthyle, 9 : 1) pour conduire au composé du titre sous la forme d'une huile brune.

### Stade C : 1-[3-(Benzyloxy)propyl]-5-méthoxy-1,3-dihydro-2H-pyrrolo[3,2-b] pyridin-2-one

Sous argon et à température ambiante, 1,10 g (16,8 mmol) de zinc en poudre sont ajoutés portion par portion à 789 mg (1,68 mmol) du composé obtenu au stade B dissous dans 15 ml d'acide acétique. Après 5 heures d'agitation à température ambiante, le milieu réactionnel est filtré sur büchner, évaporé à sec puis coévaporé en présence de toluène. Le résidu repris par l'acétate d'éthyle est lavé plusieurs fois par l'eau. La phase organique est séchée sur sulfate de magnésium, filtrée et concentrée puis purifiée par chromatographie flash sur gel de silice (éluant : éther de pétrole/acétate d'éthyle, 1 : 1) pour conduire au composé du titre sous la forme d'une huile orange.

### Stade D : 1-(3-Hydroxypropyl)-5-méthoxy-1,3-dihydro-2H-pyrrolo[3,2-b]pyridin-2-one

Sous une pression de 45 psi d'hydrogène, 510 mg (1,63 mmol) du composé obtenu au stade C et 72 mg de palladium sur charbon en suspension dans 10 ml de méthanol sont agités pendant 15 heures à température ambiante. Après filtration sur célite et évaporation, le résidu est hydrolysé par de l'eau puis extrait par l'acétate d'éthyle. La phase organique est séchée sur sulfate de magnésium et évaporée pour conduire au composé du titre sous la forme d'un solide brun.
Point de fusion : 121-122°C

### Stade E : 1-(3-Bromopropyl)-5-méthoxy-1,3-dihydro-2H-pyrrolo[3,2-b]pyridin-2-one

Sous argon et en milieu anhydre, 1,50 g (3,76 mmol) de 1,2-*bis*-diphénylphosphinoéthane sont ajoutés à 0°C à une solution de 1,25 g (3,76 mmol) de tétrabromure de carbone dans 20 ml de dichlorométhane. Après 10 minutes à 0°C, 417 mg (1,88 mmol) du composé obtenu au stade D dissous dans 10 ml de dichlorométhane sont additionnés au mélange. Le milieu est agité 30 minutes à 0°C, puis 3 heures à température ambiante. Après hydrolyse du mélange réactionnel, la phase organique est séchée sur sulfate de magnésium, filtrée et concentrée. Une purification par chromatographie flash sur gel de silice (éluant : éther de pétrole/acétate d'éthyle, 8 : 2) conduit au composé du titre sous la forme d'un solide blanc. Point de fusion : 82-83°C

### Stade F : 8-Méthoxy-3,4-dihydro-2H-pyrido[2',3':4,5]pyrrolo[2,1-b][1,3]oxazine

Sous atmosphère inerte, 160 mg (4 mmol) d'hydrure de sodium (60 % dans l'huile) sont lentement additionnés à 0°C, à une solution de 380 mg (1,34 mmol) du composé obtenu au stade E dans 10 ml de *N,N*-diméthylformamide. Le milieu réactionnel est maintenu sous agitation à 0°C pendant 30 minutes avant d'être hydrolysé par de la glace puis extrait par l'acétate d'éthyle. La phase organique est lavée plusieurs fois à l'eau, séchée sur sulfate de magnésium, concentrée et purifiée par chromatographie flash sur gel de silice (éluant : acétate d'éthyle/éther de pétrole, 95 : 5) pour conduire au composé du titre sous la forme d'un solide brun.
Point de fusion : 92-93°C

### Stade G : 8-Méthoxy-3,4-dihydro-2H-pyrido[2',3':4,5]pyrrolo[2,1-b][1,3]oxazine-10-carbaldéhyde

Sous argon et en milieu anhydre, 216 µl (2,35 mmol) d'oxychlorure de phosphore sont ajoutés goutte à goutte à 3,5 ml de *N,N*-diméthylformamide maintenus à 0°C; après 15 minutes d'agitation, 320 mg (1,56 mmol) du composé obtenu au stade G en solution dans 5 ml de *N,N*-diméthylformanide sont additionnés par transfert. La réaction est agitée 30 minutes à 0°C puis 2 heures à température ambiante. Le mélange réactionnel est évaporé à sec puis repris dans l'eau ; la phase aqueuse est alcalinisée par une solution de soude 6 N et extraite plusieurs fois par l'acétate d'éthyle. Les phases organiques réunies sont séchées sur sulfate de magnésium et évaporées pour conduire au composé du titre sous la forme d'un solide brun.
Point de fusion : 224-225°C

### Stade H : 8-Méthoxy-10-[2-nitroéthényl]-3,4-dihydro-2H-pyrido[2',3':4,5] pyrrolo[2,1-b][1,3]oxazine

300 mg (1,29 mmol) du composé obtenu au stade G dissous dans 15 ml de nitrométhane sont chauffés à 120°C pendant 4 heures en présence de 250 mg (3,23 mmol) d'acétate d'ammonium. Après retour à température ambiante et évaporation à sec, la réaction est diluée dans le dichlorométhane et lavée avec de l'eau. Après séchage sur sulfate de magnésium, l'évaporation de la phase organique conduit au composé du titre sous la forme d'un solide jaune.
Point de fusion : 248-249°C

### Stade I: 8-Méthoxy-10-(2-nitroéthyl)-3,4-dihydro-2H-pyrido[2',3':4,5] pyrrolo[2,1-b][1,3]oxazine

Sous argon et en milieu anhydre, 340 mg (1,23 mmol) du dérivé obtenu au stade H sont mis en suspension dans 7 ml d'isopropanol et 21 ml de chloroforme en présence de 810 mg de silice 230-400 mesh ; 234 mg (6,18 mmol) de borohydrure de sodium sont lentement ajoutés. Après 2 heures d'agitation à température ambiante, de l'acide acétique est ajouté et la réaction est filtrée sur verre fritté. Après évaporation des solvants, le résidu est dilué dans le dichlorométhane et lavé avec de l'eau. La phase organique est ensuite séchée sur sulfate de magnésium, filtrée, concentrée et purifiée par chromatographie flash sur gel de silice (éluant : acétate d'éthyle/éther de pétrole, 6 : 4) pour conduire au composé du titre sous la forme d'un solide jaune.
Point de fusion : 104-105°C

### Stade J : 2-(8-Méthoxy-3,4-dihydro-2H-pyrido[2',3':4,5]pyrrolo[2,1-b][1,3] oxazin-10-yl)éthylamine

Sous une pression de 55 psi d'hydrogène, 410 mg (1,48 mmol) du composé obtenu au stade I et 165 mg de nickel de Raney sont agités dans 20 ml de méthanol à 60°C pendant 15 heures. Après retour à température ambiante, la réaction est filtrée sur büchner puis évaporée pour conduire à l'amine du titre sous la forme d'une huile brune.
Point de fusion (oxalate): 84-85°C

### Préparation 15: 2-(8-Méthoxy-3,4-dihydro-2H-pyrido[4',3':4,5]pyrrolo[2,1-b][1,3] oxazin-10-yl)éthylamine

On procède comme dans la Préparation 14 à partir de la 5-méthoxy-1*H*-pyrrolo [2,3-*c*]pyridine.

### Préparation 16: 2-(2-Méthoxy-6,7,8,9 -tétrahydropyrido[2',3':4,5]pyrrolo [2,1-b] oxazépin-11-yl)éthylamine

On procède comme dans la Préparation 14 en remplaçant au stade A le 1-benzyloxy-3-bromopropane par le 1-benzyloxy-4-bromobutane.

### Préparation 17: 2-(8-Méthoxy-3,4-dihydro-2H-pyrido[3',2':4,5]pyrrolo [2,1-b][1,3] oxazin-10-yl)éthylamine

### Stade A : 1-[3-(Benzyloxy)propyl]-5-bromo-1H-pyrrolo[2,3-b]pyridine

On procède comme dans le stade A de la Préparation 14 à partir du composé obtenu au stade C de la Préparation 9.

### Stade B : 1-[3-(Benzyloxy)propyl]-5-méthoxy-1H-pyrrolo[2,3-b]pyridine

Sous argon et en milieu anhydre, 6,1 g (17,67 mmol) du composé obtenu au stade A sont dissous dans un mélange de 115 ml de *N,N*-diméthylformamide et 110 ml de méthanol ; 24 g (0,44 mole) de méthanolate de sodium et 2,53 g (17,67 mmol) de bromure cuivreux sont ajoutés à température ambiante. Le mélange est chauffé à reflux pendant 4 heures. Après évaporation des solvants, le résidu est repris dans l'acétate d'éthyle et filtré sur célite. Le filtrat est lavé avec de l'eau. La phase organique est séchée sur sulfate de magnésium, concentrée et purifiée par chromatographie flash sur gel de silice (éluant : éther de pétrole/acétate d'éthyle, 7 : 3) pour conduire au composé du titre sous la forme d'une huile jaune.

### Stade C : 2 -(8 -Méthoxy-3,4-dihydro-2H-pyrido[3',2':4,5]pyrrolo[2,1-b][1,3] oxazin-10-yl)éthylamine

On procède comme dans les stades B, C, D, E, F, G, H, I et J de la Préparation 14 à partir du composé obtenu au stade B. Huile brune.

### Préparation 18 : 11-(2-Aminoéthyl)-2-méthoxy-6H-pyrido[2',3':4,5]pyrrolo[2,1-a] isoindol-6-one

### Stade A : 5-Méthoxy-1H-pyrrolo[3,2-b]pyridine-3-carbaldéhyde

On procède comme au stade E de la Préparation 1, à partir du composé obtenu au stade B de la Préparation 1.

### Stade B : 1-(2-Iodobenzoyl)-5-méthoxy-1H-pyrrolo[3,2-b]pyridine-3-carbaldéhyde

Sous atmosphère d'argon, 0,56 mmol du composé obtenu au stade A sont dissoutes dans 5 ml de DMF puis 0,68 mmol de NaH sont additionnées à 0°C sur une période de 30 minutes. Après 30 minutes d'agitation à 0°C, 0,68 mmol de chlorure de 2-iodobenzoyle dissoutes dans 5 ml de DMF sont additionnées goutte à goutte au mélange. Après 3 heures d'agitation, les solvants sont évaporés et le résidu repris avec de l'eau et extrait avec du dichlorométhane. Après séchage sur sulfate de magnésium et évaporation des solvants, le résidu est purifié par chromatographie flash sur gel de silice (éluant: éther de pétrole/acétate d'éthyle) pour conduire au composé du titre.

### Stade C : 2-Méthoxy-6-oxo-6H-pyrido[2',3':4,5]pyrrolo[2,1-a]isoindole-11-carbaldéhyde

Sous atmosphère d'argon, 0,49 mmol du composé obtenu au stade B, 0,2 mmol de tétrakis(triphénylphosphine)palladium(0) et 0,49 mmol d'acétate de potassium dans 15 ml de DMF sont chauffés à 140°C pendant 3 heures. Après retour à température ambiante, le milieu réactionnel est filtré sur Büchner puis évaporé à sec. Le résidu est ensuite hydrolysé puis extrait par de l'acétate d'éthyle. La phase organique est séchée sur sulfate de magnésium et concentrée, le résidu est purifié par chromatographie flash sur gel de silice (éluant : éther de pétrole/acétate d'éthyle) pour conduire au composé du titre.

### Stade D : 11-(2-Aminoéthyl) -2-méthoxy-6H-pyrido[2',3 ':4.5]pyrrolo[2,1-a] isoindol-6-one

On procède comme dans les stades F-H de la Préparation 1.

### Exemple 1 : N-[2-(2-Méthoxy-6H-pyrido[2',3':4,5]pyrrolo[2,1-a]isoindol-11-yl) éthyl]acétamide

Sous argon et en milieu anhydre, 470 mg (1,68 mmol) du composé obtenu dans la Préparation 1 sont mis en solution dans 15 ml de dichlorométhane et 350 µl de pyridine ; à 0°C, 175 µl (1,85 mmol) d'anhydride acétique sont ajoutés. La réaction est agitée 3 heures à température ambiante avant d'être hydrolysée par de l'eau. La phase aqueuse est neutralisée par une solution saturée d'hydrogénocarbonate de sodium et extraite par le dichlorométhane. Après séchage sur sulfate de magnésium et évaporation des solvants, le résidu est purifié par chromatographie flash sur gel de silice (éluant : acétate d'éthyle/éther de pétrole, 8 : 2) ; après lavage par un mélange pentane/éther diéthylique, le composé du titre est obtenu sous la forme d'un solide blanc.
Point de fusion : 96-97°C

| Microanalyse élémentaire : | | | |
|---|---|---|---|
| % | C | H | N |
| Calculé | 71,01 | 5,96 | 13,07 |
| Trouvé | 70,59 | 5,74 | 12,59 |

### Exemple 2 : N-[2-(2-Méthoxy-6H-pyrido[2',3':4,5]pyrrolo[2,1-a]isoindol-11-yl) éthyl]-2-furamide

On procède comme dans l'Exemple 1 en remplaçant l'anhydride acétique par le chlorure de 2-furoyle. Solide blanc.
Point de fusion : 143-144°C

| Microanalyse élémentaire : | | | |
|---|---|---|---|
| % | C | H | N |
| Calculé | 70,76 | 5,13 | 11,25 |
| Trouvé | 70,54 | 5,10 | 10,80 |

### Exemple 3 : N-[2-(2-Méthoxy-6H-pyrido[2',3':4,5]pyrrolo[2,1-a]isoindol-11-yl) éthyl]benzamide

On procède comme dans l'Exemple 1 en remplaçant l'anhydride acétique par le chlorure de benzoyle.
Point de fusion : 177-178°C

| Microanalyse élémentaire : | | | |
|---|---|---|---|
| % | C | H | N |
| Calculé | 75,18 | 5,52 | 10,96 |
| Trouvé | 74,81 | 5,41 | 10,67 |

### Exemple 4 : N-[2-(2-Méthoxy-6H-pyrido[2',3':4,5]pyrrolo[2,1-a]isoindol-11-yl) éthyl]cyclopropane carboxamide

On procède comme dans l'Exemple 1 en remplaçant l'anhydride acétique par le chlorure de cyclopropane carbonyle.

### Exemple 5 : N-[2-(2-Méthoxy-6H-pyrido[2',3':4,5]pyrrolo[2,1-a]isoindol-11-yl) éthyl]-N'-méthylurée

On procède comme dans l'Exemple 1 en remplaçant l'anhydride acétique par l'isocyanate de méthyle.

### Exemple 6 : N-{2-[2-Méthoxy-8-(trifluorométhyl)-6H-pyrido[2',3':4,5]pyrrolo[2,1-a] isoindol-11-yl]éthyl}acétamide

On procède comme dans l'Exemple 1 à partir du composé obtenu dans la Préparation 2.

### Exemple 7 : 4-(2-Méthoxy-6H-pyrido[2',3':4,5]pyrrolo[2,1-a]isoindol-11-yl)-N-méthylbutanamide

Le produit du titre est obtenu par condensation de la *N*-méthylamine sur le composé obtenu dans la Préparation 3 après transformation en chlorure d'acide correspondant.

### Exemple 8 : N-[2-(10-Méthoxy-5,6-dihydropyrido[2',3':4,5]pyrrolo[2,1-a] isoquinolin-12-yl)éthyl]-2-furamide

On procède comme pour l'Exemple 2 à partir du composé obtenu dans la Préparation 4.
Point de fusion : 188-189°C

| Microanalyse élémentaire : | | | |
|---|---|---|---|
| % | C | H | N |
| Calculé | 71,30 | 5,46 | 10,85 |
| Trouvé | 70,65 | 5,44 | 10,63 |

### Exemple 9 : N-[2-(10-Méthoxy-5,6-dihydropyrido[2',3':4,5]pyrrolo[2,1-a] isoquinolin-12-yl)éthyl]-2-phénylacétamide

On procède comme dans l'Exemple 8 en remplaçant le chlorure de 2-furoyle par le chlorure de benzoyle.

### Exemple 10 : N-[2-(11-Méthoxy-6,7-dihydro-5H-pyrido[2',3':4,5]pyrrolo[2,1-α][2] benzazépin-13-yl)éthyl]acétamide

Sous argon et en milieu anhydre, 550 mg (1,79 mmol) du composé obtenu dans la Préparation 5 sont mis en solution dans 15 ml de dichlorométhane et 372 µl de pyridine ; à 0°C, 186 µl (1,97 mmol) d'anhydride acétique sont ajoutés. La réaction est agitée 5 heures à température ambiante avant d'être hydrolysée par de l'eau. La phase aqueuse est neutralisée par une solution saturée d'hydrogénocarbonate de sodium et extraite par le dichlorométhane. Après séchage sur sulfate de magnésium et évaporation des solvants, le résidu est purifié par chromatographie flash sur gel de silice (éluant : dichlorométhane/méthanol, 99: 1); après lavage par un mélange pentane/éther diéthylique, le composé du titre est obtenu sous la forme d'un solide blanc.
Point de fusion : 73-74°C

| Microanalyse élémentaire : | | | |
|---|---|---|---|
| % | C | H | N |
| Calculé | 72,12 | 6,63 | 12,03 |
| Trouvé | 71,99 | 6,67 | 11,56 |

### Exemple 11 : N-[2-(11-Méthoxy-6,7-dihydro-5H-pyrido[2',3':4,5]pyrrolo[2,1-a][2] benzazépin-13-yl)éthyl]-2-furamide

Sous argon et en milieu anhydre, 730 mg (2,38 mmol) du composé obtenu dans la Préparation 5 sont mis en solution dans 10 ml de dichlorométhane et 1 ml (7,13 mmol) de triéthylamine ; à 0°C, 330 µl (3,33 mmol) de chlorure de 2-furoyle sont ajoutés. La réaction est agitée pendant 15 heures à température ambiante avant d'être hydrolysée par de l'eau puis extraite par le dichlorométhane. Après séchage sur sulfate de magnésium et évaporation des solvants, le résidu est purifié par chromatographie flash sur gel de silice (éluant : éther de pétrole/acétate d'éthyle, 6: 4) puis par lavage par un mélange hexane/éther de pétrole pour conduire au composé du titre sous la forme d'un solide blanc.
Point de fusion : 70-71°C

| Microanalyse élémentaire : | | | |
|---|---|---|---|
| % | C | H | N |
| Calculé | 71,80 | 5,88 | 10,21 |
| Trouvé | 71,39 | 5,77 | 10,47 |

### Exemple 12 : N-[2-(11-Hydroxy-6,7-dihydro-5H-pyrido[2',3':4,5]pyrrolo[2,1-a][2] benzazépin-13-yl)éthyl]-2-furamide

Sous atmosphère inerte, 582 mg (4,36 mmol) de chlorure d'aluminium sont additionnés à une solution de 350 mg (0,87 mmol) du composé de l'Exemple 11 dans 15 ml de dichlorométhane. Le milieu réactionnel est porté à reflux pendant 6 heures avant d'être hydrolysé par de l'eau. La phase aqueuse est neutralisée par une solution d'hydrogénocarbonate de sodium et extraite par le dichlorométhane. Après séchage sur sulfate de magnésium et évaporation des solvants, le résidu est purifié par chromatographie flash sur gel de silice (éluant : acétate d'éthyle/méthanol, 95 : 5) puis par lavage par un mélange hexane/éther diéthylique pour conduire au composé du titre sous la forme d'un solide blanc.
Point de fusion : > 260°C

### Exemple 13 : N-Cyclobutyl-4-(6,7-dihydro-5H-pyrido[2',3':4,5]pyrrolo[2,1-a][2] benzazépin-13-yl)butanamide

On procède comme dans l'Exemple 7, à partir du composé obtenu dans la Préparation 6 et en remplaçant la *N*-méthylamine par la *N*-cyclobutylamine.

### Exemple 14 : N-[2-(11-Ethyl-6,7-dihydro-5H-pyrido[2',3':4,5]pyrrolo[2,1-a] benzazépin-13-yl)éthyl]acétamide

On procède comme dans l'Exemple 10 à partir du composé obtenu dans la Préparation 7.

### Example 15 : N-[2-(2-Méthoxy-6,7,8,9-tétrahydropyrido[2,3-b]indolizin-10-yl)éthyl] acétamide

Sous argon et en milieu anhydre, 367 mg (1,49 mmol) du composé obtenu dans la Préparation 8 sont mis en solution dans 15 ml de dichlorométhane et 320 µl de pyridine ; à 0°C, 160 µl (1,64 mmol) d'anhydride acétique sont ajoutés. La réaction est agitée 3 heures à température ambiante avant d'être hydrolysée par de l'eau. La phase aqueuse est neutralisée par une solution saturée d'hydrogénocarbonate de sodium et extraite par le dichlorométhane. Après séchage sur sulfate de magnésium et évaporation des solvants, le résidu est purifié par chromatographie flash sur gel de silice (éluant : acétate d'éthyle) ; après lavage par un mélange pentane/éther diéthylique, le composé du titre est obtenu sous la forme d'un solide blanc.
Point de fusion : 89-90°C

| Microanalyse élémentaire : | | | |
|---|---|---|---|
| % | C | H | N |
| Calculé | 66,88 | 7,37 | 14,62 |
| Trouvé | 66,12 | 7,10 | 14,57 |

### Exemple 16 : N-[2-(2-Méthoxy-6,7,8,9-tétrahydropyrido[2,3-b]indolizin-10-yl)éthyl] -2-furamide

Sous argon et en milieu anhydre, 310 mg (1,27 mmol) du composé obtenu dans la Préparation 8 sont mis en solution dans 15 ml de dichlorométhane et 530 µl (3,79 mmol) de triéthylamine ; à 0°C, 176 µl (1,77 mmol) de chlorure de 2-furoyle sont ajoutés. La réaction est agitée pendant 3 heures à température ambiante avant d'être hydrolysée par de l'eau puis extraite par le dichlorométhane. Après séchage sur sulfate de magnésium et évaporation des solvants, le résidu est purifié par chromatographie flash sur gel de silice (éluant : éther de pétrole/acétate d'éthyle, 1: 1) puis par lavage par un mélange hexane/éther de pétrole pour conduire au composé du titre sous la forme d'un solide blanc.
Point de fusion : 104-105°C

| Microanalyse élémentaire : | | | |
|---|---|---|---|
| % | C | H | N |
| Calculé | 70,76 | 5,13 | 11,25 |
| Trouvé | 70,54 | 5,10 | 10,80 |

### Exemple 17 : N-[2-(3-Méthoxy-6,7,8,9-tétrahydropyrido[2,3-b]indolizin-5-yl)éthyl] acétamide

Sous argon et en milieu anhydre, 262 mg (1,07 mmol) du composé obtenu dans la Préparation 9 sont mis en solution dans 10 ml de dichlorométhane et 220 µl de pyridine ; à 0°C, 110 µl (1,18 mmol) d'anhydride acétique sont ajoutés. La réaction est agitée 3 heures à température ambiante avant d'être hydrolysée par de l'eau. La phase aqueuse est neutralisée par une solution saturée d'hydrogénocarbonate de sodium et extraite par le dichlorométhane. Après séchage sur sulfate de magnésium et évaporation des solvants, le résidu est purifié par chromatographie flash sur gel de silice (éluant : acétate d'éthyle) ; après lavage par un mélange pentane/éther diéthylique, le composé du titre est obtenu sous la forme d'un solide blanc.
Point de fusion : 148-149°C

| Microanalyse élémentaire : | | | |
|---|---|---|---|
| % | C | H | N |
| Calculé | 66,88 | 7,37 | 14,62 |
| Trouvé | 66,70 | 7,37 | 14,18 |

### Exemple 18 : N-[2-(2-Méthoxy-6,7,8,9-tétrahydropyrido[2,3-b]indolizin-10-yl)éthyl] -N'-phénylurée

On procède comme dans l'Exemple 5 à partir du composé obtenu dans la Préparation 8 en remplaçant l'isocyanate de méthyle par l'isocyanate de phényle.

### Exemple 19 : N-Cyclopropyl-4-(2-méthoxy-6,7,8,9-tétrahydropyrido[2,3-b]indolizin-10-yl)butanamide

On procède comme dans l'Exemple 7, à partir du composé obtenu dans la Préparation 10 et en remplaçant la *N*-méthylamine par la *N*-cyclopropylamine.

### Exemple 20 : N-[2-(2-Méthoxy-7,8-dihydro-6H-pyrido[2,3-b]pyrrolizin-9-yl)éthyl] acétamide

On procède comme dans l'Exemple 15 à partir du composé obtenu dans la Préparation 11.

### Exemple 21 : N-[2-(2-Isopropyl-7,8-dihydro-6H-pyrido[2,3-b]pyrrolizin-9-yl)éthyl] acétamide

On procède comme dans l'Exemple 15 à partir du composé obtenu dans la Préparation 12.

### Exemple 22 : N-{2-[2-(Benzyloxy)-7,8,9,10-tétrahydro-6H-pyrido[2',3':4,5]pyrrolo [1,2-a]azépin-11-yl]éthyl}heptanamide

On procède comme dans l'Exemple 1 à partir du composé obtenu dans la Préparation 13 en remplaçant l'anhydride acétique par le chlorure d'heptanoyle.

### Exemple 23 : N-[2-(8-Méthoxy-3,4-dihydro-2H-pyrido[2',3':4,5]pyrrolo[2,1-b][1,3] oxazin-10-yl)éthyl]acétamide

Sous argon et en milieu anhydre, 350 mg (1,41 mmol) du composé obtenu dans la Préparation 14 sont mis en solution dans 12 ml de dichlorométhane et 300 µl de pyridine ; à 0°C, 150 µl (1,56 mmol) d'anhydride acétique sont ajoutés. La réaction est agitée 6 heures à température ambiante avant d'être hydrolysée par de l'eau. La phase aqueuse est neutralisée par une solution saturée d'hydrogénocarbonate de sodium et extraite par le dichlorométhane. Après séchage sur sulfate de magnésium et évaporation des solvants, le résidu est purifié par chromatographie flash sur gel de silice (éluant : acétate d'éthyle) ; après lavage par un mélange pentane/éther diéthylique, le composé du titre est obtenu sous la forme d'un solide blanc.
Point de fusion : 92-93°C

| Microanalyse élémentaire : | | | |
|---|---|---|---|
| % | C | H | N |
| Calculé | 62,27 | 6,62 | 14,52 |
| Trouvé | 62,02 | 6,66 | 14,40 |

### Exemple 24 : N-[2-(8-Méthoxy-3,4-dihydro-2H-pyrido[2',3':4,5]pyrrolo[2,1-b][1,3] oxazin-10-yl)éthyl]-2-furamide

Sous argon et en milieu anhydre, 200 mg (0,81 mmol) du composé obtenu dans la Préparation 14 sont mis en solution dans 6 ml de dichlorométhane et 340 µl (2,43 mmol) de triéthylamine ; à 0°C, 115 µl (1,13 mmol) de chlorure de 2-furoyle sont ajoutés. La réaction est agitée pendant 15 heures à température ambiante avant d'être hydrolysée par de l'eau puis extraite par le dichlorométhane. Après séchage sur sulfate de magnésium et évaporation des solvants, le résidu est purifié par chromatographie flash sur gel de silice (éluant : éther de pétrole/acétate d'éthyle, 1 : 1) puis par lavage par un mélange hexane/éther de pétrole pour conduire au composé du titre sous la forme d'un solide blanc.
Point de fusion : 139-140°C

| Microanalyse élémentaire : | | | |
|---|---|---|---|
| % | C | H | N |
| Calculé | 63,33 | 5,61 | 12,31 |
| Trouvé | 63,52 | 5,64 | 11,93 |

### Exemple 25 : N-[2-(8-Méthoxy-3,4-dihydro-2H-pyrido[4',3':4,5]pyrrolo[2,1-b][1,3] oxazin-10-yl)éthyl]pentanamide

On procède comme dans l'Exemple 23 à partir du composé obtenu dans la Préparation 15 en remplaçant l'anhydride acétique par le chlorure de pentanoyle.

### Exemple 26 : N-[2-(2-Méthoxy-6,7,8,9-tétrahydropyrido[2',3':4,5]pyrrolo[2,1-b][1,3] oxazépin-11-yl)éthyl]acétamide

On procède comme dans l'Exemple 23 à partir du composé obtenu dans la Préparation 16.

### Exemple 27 : N-[2-(8-Méthoxy-3,4-dihydro-2H-pyrido[3',2':4,5]pyrrolo[2,1-b][1,3] oxazin-10-yl)éthyl]-2-furamide

Sous argon et en milieu anhydre, 218 mg (0,88 mmol) du composé obtenu dans la Préparation 17 sont mis en solution dans 10 ml de dichlorométhane et 370 µl (2,64 mmol) de triéthylamine ; à 0°C, 122 µl (1,23 mmol) de chlorure de 2-furoyle sont ajoutés. La réaction est agitée pendant 3 heures à température ambiante avant d'être hydrolysée par de l'eau puis extraite par le dichlorométhane. Après séchage sur sulfate de magnésium et évaporation des solvants, le résidu est purifié par chromatographie flash sur gel de silice (éluant: éther de pétrole/acétate d'éthyle, 1: 1) puis par lavage par un mélange hexane/éther de pétrole pour conduire au composé du titre sous la forme d'un solide blanc.
Point de fusion : 70-71 °C

### Exemple 28 : 2-Iodo-N-[2-(2-méthoxy-6H-pyrido[2',3':4,5]pyrrolo[2,1-a]isoindol-11-yl)éthyl]benzamide

Sous atmosphère d'argon, 1,14 mmol du composé obtenu dans la Préparation 1 sont dissous dans 10 ml de dichlorométhane et 3,43 mmol de triéthylamine. A 0°C, 1,60 mmol de chlorure de 2-iodobenzoyle dissous dans 5 ml de dichlorométhane sont additionnés goutte à goutte au mélange. La réaction est agitée pendant 18 heures à température ambiante avant d'être hydrolysée par de l'eau, puis extraite par du dichlorométhane. Après séchage sur sulfate de magnésium et évaporation des solvants, le résidu est purifié par chromatographie flash sur gel de silice (éluant : éther de pétrole/acétate d'éthyle, 1/1) pour conduire au composé du titre sous la forme d'un solide blanc.
Point de fusion : 224-225°C

### Exemple 29 : N-[2-(2-Méthoxy-6-oxo-6H-pyrido[2',3':4,5]pyrrolo[2,1-a]isoindol-11-yl)éthyl]-2-furamide

Sous atmosphère d'argon, 1 mmol du composé obtenu dans la Préparation 18 est mise en solution dans 10 ml de dichlorométhane et 3 mmol de triéthylamine puis 1,4 mmol de chlorure de 2-furoyle sont ajoutées goutte à goutte à 0°C. La réaction est agitée pendant 18 heures avant d'être hydrolysée par de l'eau, puis extraite par du dichlorométhane. Après séchage sur sulfate de magnésium et évaporation des solvants, le résidu est purifié par chromatographie flash sur gel de silice (éluant : éther de pétrole/acétate d'éthyle) pour conduire au composé du titre.

### Exemple 30 : N-[2-(2-Hydroxy-6,7,8,9-tétrahydropyrido[2,3-b]indolizin-10-yl)éthyl]-2-furamide

Sous argon et en milieu anhydre, 0,884 mmol du composé obtenu dans l'Exemple 16 sont dissoutes dans 16 ml de dichlorométhane puis 4,42 mmol de chlorure d'aluminium sont ajoutés. Le milieu réactionnel est agité à reflux pendant 18 heures. La réaction est hydrolysée, puis neutralisée par une solution d'hydrogénocarbonate de sodium. Après une extraction au dichlorométhane, les phases organiques sont séchées sur sulfate de magnésium et évaporées sous vide. Le résidu obtenu est lavé par de l'hexane et de l'éther diéthylique pour conduire au produit du titre.
Point de fusion : 270°C

| Microanalyse élémentaire : | | | |
|---|---|---|---|
| % | C | H | N |
| Calculé | 66,45 | 5,89 | 12,91 |
| Trouvé | 65,02 | 6,04 | 12,54 |

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la *pars tuberalis* de mouton. La *pars tuberalis* de l'adénohypophyse est en effet caractérisée, chez les mammifères, par une haute densité en récepteurs de la mélatonine (Journal of Neuroendocrinology, 1, pp. 1-4, 1989).

### Protocole

1) Les membranes de *pars tuberalis* de mouton sont préparées et utilisées comme tissu cible dans des expériences de saturation pour déterminer les capacités et affinités de liaison pour la 2-[¹²⁵I]-iodomélatonine.
2) Les membranes de *pars tuberalis* de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : 1. Etude de liaison aux récepteurs mt₁ et MT₂ de la mélatonine

Les expériences de liaison aux récepteurs mt₁ ou MT₂ sont réalisées en utilisant la 2-[¹²⁵I]-iodomélatonine comme radioligand de référence. La radioactivité retenue est déterminée à l'aide d'un compteur à scintillation liquide.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

### 2. Etude de liaison aux sites de liaisons MT₃ de la melatonine

Les expériences de liaison sur les sites *MT*_{*3*} sont réalisées sur membranes de cerveau de hamster en utilisant la 2-[¹²⁵I] iodomélatonine comme radioligand. Les membranes sont incubées pendant 30 minutes avec la 2-[¹²⁵I] iodomélatonine à la température de 4°C et différentes concentrations des composés à tester. Après l'incubation, les membranes sont rapidement filtrées puis lavées par du tampon froid à l'aide d'un système de filtration. La radioactivité fixée est mesurée par un compteur à scintillation. Les valeurs d'IC₅₀ (concentration inhibant de 50 % la liaison spécifique) sont calculées à partir des courbes de compétition selon un modèle de régression non linéaire.

Ainsi, les valeurs d'IC₅₀ trouvées pour les composés de l'invention attestent d'une liaison pour l'un ou l'autre des sous-types réceptoriels, ces valeurs étant ≤ 10 µM.

### EXEMPLE D: Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien *via* le système mélatoninergique.

### EXEMPLE E : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE F : Activité des composés de l'invention sur l'artère caudale de rat

Les composés de l'invention ont été testés *in vitro* sur l'artère caudale de rat. Les récepteurs mélatoninergiques sont présents sur ces vaisseaux ce qui en fait un modèle pharmacologique relevant pour étudier l'activité de ligands mélatoninergiques. La stimulation des récepteurs peut induire soit une vasoconstriction soit une dilatation en fonction du segment artériel étudié.

### Protocole

Des rats âgés de 1 mois sont habitués pendant 2 à 3 semaines à un cycle lumière/obscurité 12h/12h.
Après sacrifice, l'artère caudale est isolée et maintenue dans un milieu richement oxygéné. Les artères sont ensuite cannulées aux deux extrémités, suspendues verticalement dans une chambre d'organe dans un milieu approprié et perfusées via leur extrémité proximale. Les changements de pression dans le débit de la perfusion permettent d'évaluer l'effet vasoconstricteur ou vasodilatateur des composés.
L'activité des composés est évaluée sur des segments pré-contractés par la phényléphrine (1 µM). Une courbe concentration-réponse est déterminée de façon non-cumulative par addition d'une concentration du composé étudié sur le segment pré-contracté. Lorsque l'effet observé a atteint l'équilibre, le milieu est changé et la préparation laissée 20 minutes avant l'addition d'une même concentration de phényléphrine et d'une nouvelle concentration du composé étudié.

### Résultats

Les composés de l'invention modifient de façon significative le diamètre des artères caudales préconstrictées par la phényléphrine.

### EXEMPLE G : Composition pharmaceutique : Comprimés

| | |
|---|---|
| 1000 comprimés dosés à 5 mg de *N*-[2-(2-méthoxy-6*H*-pyrido[2',3':4,5]pyrrolo [2,1-*a*]isoindol-11-yl)éthyl]-2-furamide (Exemple 2) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 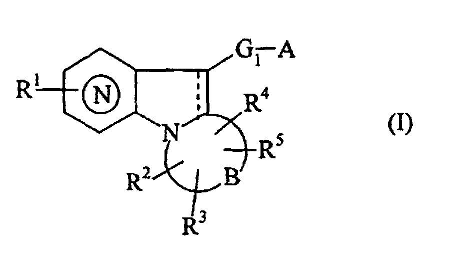 dans laquelle :
- la notation 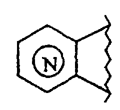 représente les groupements 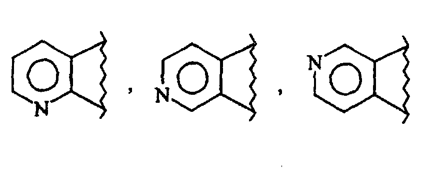 ou 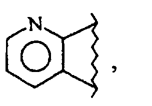
- R¹ représente un atome d'halogène ou un groupement -R, -OR, -S(O)ₙR, -NRR', 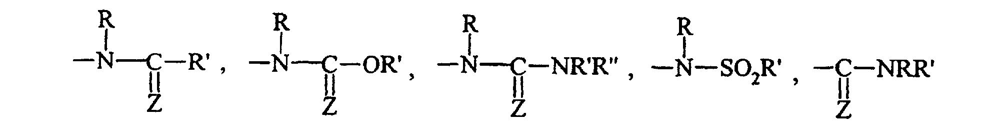 ou -SO₂NRR' (où n vaut 0, 1 ou 2, Z représente un atome de soufre ou d'oxygène, et R, R' et R", identiques ou différents, représentent un atome d'hydrogène ou un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, cycloalkyle (C₃-C₈) substitué ou non, cycloalkyl (C₃-C₈) alkyle (C₁-C₆) linéaire ou ramifié substitué ou non, aryle, arylalkyle (C₁-C₆) linéaire ou ramifié, hétéroaryle ou hétéroarylalkyle (C₁-C₆) linéaire ou ramifié,
(R et R') et (R' et R") pouvant de plus former ensemble avec l'atome d'azote qui les porte un groupement morpholinyle, piperidinyle, piperazinyle ou pyrrolidinyle, ces groupements étant non substitués ou substitués),
- G₁ représente une chaîne alkylène comportant de 1 à 4 atomes de carbone éventuellement substituée par un groupement R, OR, COR ou COOR (où R est tel que défini précédemment),
ou G₁ représente une chaîne alkylène comportant de 1 à 4 atomes de carbone dans laquelle un des groupements CH₂ peut être remplacé par un groupement cycloalkylène (C₃-C₈),
- A représente un groupement 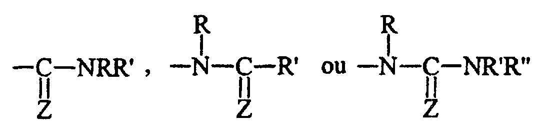 dans lesquels R, R', R" et Z sont tels que définis précédemment,
- B forme avec l'atome d'azote et l'atome de carbone auxquels il est rattaché un cycle comportant de 5 à 8 chaînons pouvant contenir une ou plusieurs insaturations et pouvant contenir, en plus de l'atome d'azote, un hétéroatome supplémentaire choisi parmi oxygène, soufre ou azote,
- R² et R³, identiques ou différents, représentent un atome d'hydrogène ou un groupement alkyle (C₁-C₆) linéaire ou ramifié, alkoxy (C₁-C₆) linéaire ou ramifié ou hydroxyle,
ou R² et R³ forment ensemble un groupement oxo,
- R⁴ et R⁵ représentent un atome d'hydrogène ou forment ensemble avec deux atomes adjacents du cycle B qui les portent un groupement choisi parmi aryle ou hétéroaryle,
- la notation ----- signifie que la liaison est simple ou double, étant entendu que la valence des atomes est respectée,
étant entendu que :
- le terme « substitué » affecté aux expressions « alkyle », « alkényle », « alkynyle », « cycloalkyle », « morpholinyle », « pipéridinyle », « pipérazinyle » et « pyrrolidinyle » signifie que ces groupements peuvent être substitués par un ou plusieurs groupements choisis parmi hydroxyle, oxo, alkoxy, alkyle, polyhalogénoalkyle, ou atomes d'halogène,
- le terme « substitué » affecté à l'expression « cycloalkylalkyle » signifie que la partie cyclique du groupement est substituée par un ou plusieurs groupements choisis parmi hydroxyle, oxo, alkoxy, alkyle, polyhalogénoalkyle, ou atomes d'halogène,
- par « aryle» on entend un groupement phényle, naphtyle ou biphényle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, alkoxycarbonyle, amido, ou atomes d'halogène,
- par « hétéroaryle » on entend un groupement furyle, pyrrolyle, imidazolyle, pyridyle, thiényle, pyrazinyle, benzothiényle, benzofurannyle, indolyle, benzimidazolyle, quinolyle ou quinazolinyle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, amido, ou atomes d'halogène,
leurs énantiomères et diastéréoisomères, ainsi que leurs sels d'addition à un acide ou à une base pharmaceutiquement acceptable.

2. Composés de formule (I) selon la revendication 1 : 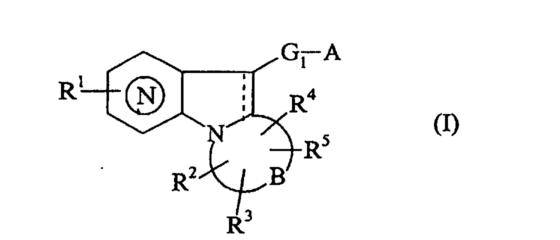 dans laquelle :
- la notation 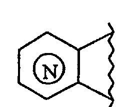 représente les groupements 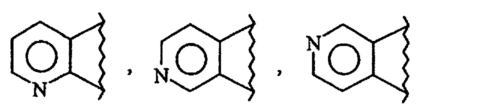 ou 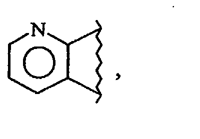
- R¹ représente un atome d'halogène ou un groupement -R, -OR, -S(O)ₙR, -NRR', 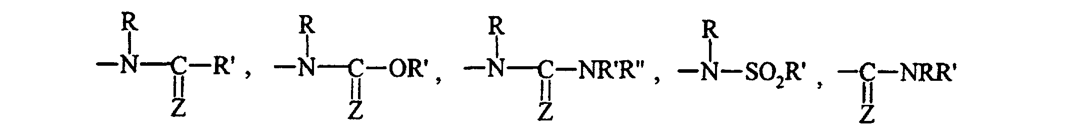 ou -SO₂NRR' (où n vaut 0, 1 ou 2, Z représente un atome de soufre ou d'oxygène, et R, R' et R", identiques ou différents, représentent un atome d'hydrogène ou un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, cycloalkyle (C₃-C₈) substitué ou non, cycloalkyl (C₃-C₈) alkyle (C₁-C₆) linéaire ou ramifié substitué ou non, aryle, arylalkyle (C₁-C₆) linéaire ou ramifié, hétéroaryle ou hétéroarylalkyle (C₁-C₆) linéaire ou ramifié,
(R et R') et (R' et R") pouvant de plus former ensemble avec l'atome d'azote qui les porte un groupement morpholinyle, piperidinyle, piperazinyle ou pyrrolidinyle, ces groupements étant non substitués ou substitués),
- G₁ représente une chaîne alkylène comportant de 1 à 4 atomes de carbone éventuellement substituée par un groupement R, OR, COR ou COOR (où R est tel que défini précédemment),
- A représente un groupement 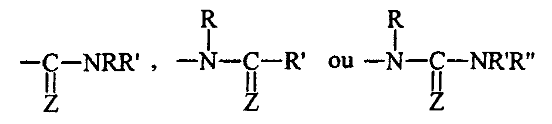 dans lesquels R, R', R" et Z sont tels que définis précédemment,
- B forme avec l'atome d'azote et l'atome de carbone auxquels il est rattaché un cycle comportant de 5 à 8 chaînons pouvant contenir une ou plusieurs insaturations et pouvant contenir, en plus de l'atome d'azote, un hétéroatome supplémentaire choisi parmi oxygène, soufre ou azote,
- R² et R³, identiques ou différents, représentent un atome d'hydrogène ou un groupement alkyle (C₁-C₆) linéaire ou ramifié, alkoxy (C₁-C₆) linéaire ou ramifié ou hydroxyle,
ou R² et R³ forment ensemble un groupement oxo,
- R⁴ et R⁵ représentent un atome d'hydrogène ou forment ensemble avec deux atomes adjacents du cycle B qui les portent un groupement choisi parmi aryle ou hétéroaryle,
- la notation ----- signifie que la liaison est simple ou double, étant entendu que la valence des atomes est respectée,
étant entendu que :
- le terme « substitué » affecté aux expressions « alkyle », « alkényle », « alkynyle », « cycloalkyle », « morpholinyle », « pipéridinyle », « pipérazinyle » et « pyrrolidinyle » signifie que ces groupements peuvent être substitués par un ou plusieurs groupements choisis parmi hydroxyle, oxo, alkoxy, alkyle, polyhalogénoalkyle, ou atomes d'halogène,
- le terme « substitué » affecté à l'expression « cycloalkylalkyle » signifie que la partie cyclique du groupement est substituée par un ou plusieurs groupements choisis parmi hydroxyle, oxo, alkoxy, alkyle, polyhalogénoalkyle, ou atomes d'halogène,
- par « aryle » on entend un groupement phényle, naphtyle ou biphényle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, alkoxycarbonyle, amido, ou atomes d'halogène,
- par « hétéroaryle » on entend un groupement furyle, pyrrolyle, imidazolyle, pyridyle, thiényle, pyrazinyle, benzothiényle, benzofurannyle, indolyle, benzimidazolyle, quinolyle ou quinazolinyle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, amido, ou atomes d'halogène,
leurs énantiomères et diastéréoisomères, ainsi que leurs sels d'addition à un acide ou à une base pharmaceutiquement acceptable.

3. Composés de formule (I) selon la revendication 1 : 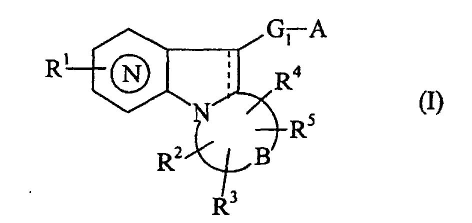 dans laquelle :
- la notation 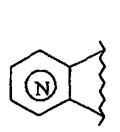 représente les groupements 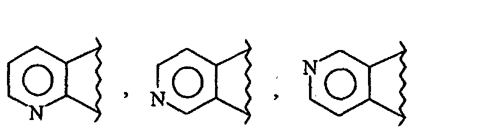 ou 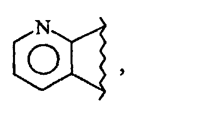
- R¹ représente un atome d'halogène ou un groupement -R, -OR, -S(O)ₙR, -NRR', 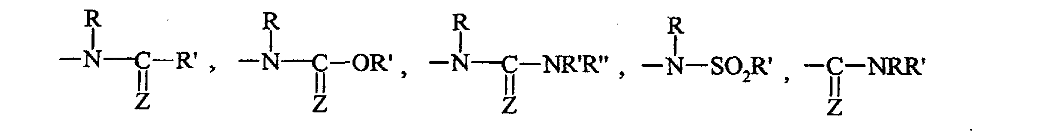 ou -SO₂NRR' (où n vaut 0, 1 ou 2, Z représente un atome de soufre ou d'oxygène, et R, R' et R", identiques ou différents, représentent un atome d'hydrogène ou un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, cycloalkyle (C₃-C₈) substitué ou non, cycloalkyl (C₃-C₈) alkyle (C₁-C₆) linéaire ou ramifié substitué ou non, aryle, arylalkyle (C₁-C₆) linéaire ou ramifié, hétéroaryle ou hétéroarylalkyle (C₁-C₆) linéaire ou ramifié,
(R et R') et (R' et R") pouvant de plus former ensemble avec l'atome d'azote qui les porte un groupement morpholinyle, piperidinyle, piperazinyle ou pyrrolidinyle, ces groupements étant non substitués ou substitués),
- G₁ représente une chaîne alkylène comportant de 1 à 4 atomes de carbone dans laquelle un des groupements CH₂ peut être remplacé par un groupement cycloalkylène (C₃-C₈),
- A représente un groupement 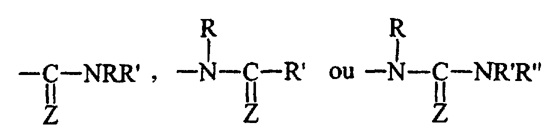 dans lesquels R, R', R" et Z sont tels que définis précédemment,
- B forme avec l'atome d'azote et l'atome de carbone auxquels il est rattaché un cycle comportant de 5 à 8 chaînons pouvant contenir une ou plusieurs insaturations et pouvant contenir, en plus de l'atome d'azote, un hétéroatome supplémentaire choisi parmi oxygène, soufre ou azote,
- R² et R³, identiques ou différents, représentent un atome d'hydrogène ou un groupement alkyle (C₁-C₆) linéaire ou ramifié, alkoxy (C₁-C₆) linéaire ou ramifié ou hydroxyle,
ou R² et R³ forment ensemble un groupement oxo,
- R⁴ et R⁵ représentent un atome d'hydrogène ou forment ensemble avec deux atomes adjacents du cycle B qui les portent un groupement choisi parmi aryle ou hétéroaryle,
- la notation ----- signifie que la liaison est simple ou double, étant entendu que la valence des atomes est respectée,
étant entendu que :
- le terme « substitué » affecté aux expressions « alkyle », « alkényle », « alkynyle », « cycloalkyle », « morpholinyle », « pipéridinyle », « pipérazinyle » et « pyrrolidinyle » signifie que ces groupements peuvent être substitués par un ou plusieurs groupements choisis parmi hydroxyle, oxo, alkoxy, alkyle, polyhalogénoalkyle, ou atomes d'halogène,
- le terme « substitué » affecté à l'expression « cycloalkylalkyle » signifie que la partie cyclique du groupement est substituée par un ou plusieurs groupements choisis parmi hydroxyle, oxo, alkoxy, alkyle, polyhalogénoalkyle, ou atomes d'halogène,
- par « aryle » on entend un groupement phényle, naphtyle ou biphényle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, alkoxycarbonyle, amido, ou atomes d'halogène,
- par « hétéroaryle » on entend un groupement furyle, pyrrolyle, imidazolyle, pyridyle, thiényle, pyrazinyle, benzothiényle, benzofurannyle, indolyle, benzimidazolyle, quinolyle ou quinazolinyle, ces groupements étant non substitués ou substitués par un ou plusieurs groupements, identiques ou différents, choisis parmi hydroxyle, alkoxy, alkyle, amino, alkylamino, dialkylamino, nitro, cyano, polyhalogénoalkyle, formyle, carboxy, amido, ou atomes d'halogène,
leurs énantiomères et diastéréoisomères, ainsi que leurs sels d'addition à un acide ou à une base pharmaceutiquement acceptable.

4. Composés de formule (I) selon la revendication 1 pour lesquels B forme avec l'atome d'azote et l'atome de carbone auxquels il est rattaché un cycle pyrrolidine, pipéridine, (perhydro)azépine ou (perhydro)azocine, leurs énantiomères et diastéréoisomères, ainsi que leurs sels d'addition à un acide ou à une base pharmaceutiquement acceptable.

5. Composés de formule (I) selon la revendication 1 pour lesquels B forme avec l'atome d'azote et l'atome de carbone auxquels il est rattaché un cycle 1,3-(perhydro)oxazine, leurs énantiomères et diastéréoisomères, ainsi que leurs sels d'addition à un acide ou à une base pharmaceutiquement acceptable.

6. Composés de formule (I) selon la revendication I pour lesquels R², R³, R⁴ et R⁵ représentent simultanément un atome d'hydrogène, leurs énantiomères et diastéréoisomères, ainsi que leurs sels d'addition à un acide ou à une base pharmaceutiquement acceptable.

7. Composés de formule (I) selon la revendication 1 pour lesquels R² et R³ représentent un atome d'hydrogène et R⁴ et R⁵ forment ensemble avec les carbones adjacents qui les portent un groupement phényle, leurs énantiomères et diastéréoisomères, ainsi que leurs sels d'addition à un acide ou à une base pharmaceutiquement acceptable.

8. Composés de formule (I) selon la revendication 1 pour lesquels R¹ représente un groupement OR, leurs énantiomères et diastéréoisomères, ainsi que leurs sels d'addition à un acide ou à une base pharmaceutiquement acceptable.

9. Composés de formule (I) selon la revendication 1 pour lesquels G₁ représente un groupement (CH₂)ₚ où p vaut 2 ou 3, leurs énantiomères et diastéréoisomères, ainsi que leurs sels d'addition à un acide ou à une base pharmaceutiquement acceptable.

10. Composés de formule (I) selon la revendication 1 pour lesquels A représente un groupement NHCOR, leurs énantiomères et diastéréoisomères, ainsi que leurs sels d'addition à un acide ou à une base pharmaceutiquement acceptable.

11. Composés de formule (I) selon la revendication 1 pour lesquels A représente un groupement CONHR, leurs énantiomères et diastéréoisomères, ainsi que leurs sels d'addition à un acide ou à une base pharmaceutiquement acceptable.

12. Composés de formule (I) selon la revendication 1 qui sont le *N*-[2-(2-méthoxy-6*H*-pyrido[2',3':4,5]pyrrolo[2,1-*a*]isoindol-11-yl)éthyl]acétamide, le *N*-[2-(2-méthoxy-6*H*-pyrido[2',3':4,5]pyrrolo[2,1-*a*]isoindol-11-yl)éthyl]-2-furamide, le *N*-[2-(2-méthoxy-6*H*-pyrido[2',3':4,5]pyrrolo[2,1-*a*]isoindol-11-yl)éthyl]benzamide, le 2-iodo-*N*-[2-(2-méthoxy-6*H*-pyrido[2',3':4,5]pyrrolo[2,1-*a*]isoindol-11-yl)éthyl]benzamide ainsi que leurs sels d'addition à un acide ou à une base pharmaceutiquement acceptable.

13. Composés de formule (I) selon la revendication 1 qui sont le *N*-[2-(3-méthoxy-6,7,8,9-tetrahydropyrido[3,2-*b*]indolizin-5-yl)éthyl]acétamide, le *N*-[2-(2-méthoxy-6,7,8,9-tetrahydropyrido[2,3-*b*]indolizin-10-yl)éthyl]-2-furamide, le *N*-[2-(2-méthoxy-6,7,8,9-tetrahydropyrido[2,3-*b*]indolizin-10-yl)éthyl]acétamide, le *N*-[2-(2-hydroxy-6,7,8,9-tétrahydropyrido[2,3-*b*]indolizin-10-yl)éthyl]-2-furamide ainsi que leurs sels d'addition à un acide ou à une base pharmaceutiquement acceptable.

14. Composés de formule (I) selon la revendication 1 qui sont le *N*-[2-(8-méthoxy-3,4-dihydro-2*H*-pyrido[2',3':4,5]pyrrolo[2,1-*b*][1,3]oxazin-10-yl)éthyl]acétamide, le *N*-[2-(8-méthoxy-3,4-dihydro-2*H*-pyrido[2',3':4,5]pyrrolo[2,1-*b*][1,3]oxazin-10-yl)éthyl]-2-furamide ainsi que leurs sels d'addition à un acide ou à une base pharmaceutiquement acceptable.

15. Composé de formule (I) selon la revendication 1 qui est le *N*-[2-(10-Méthoxy-5,6-dihydro-pyrido[2',3':4,5]pyrrolo[2,1-*a*]isoquinolin-12-yl)éthyl]-2-furamide ainsi que ses sels d'addition à un acide ou à une base pharmaceutiquement acceptable.

16. Composés de formule (I) selon la revendication 1 qui sont le *N*-[2-(11-méthoxy-6,7-dihydro-5*H*-pyrido[2',3':4,5]pyrrolo[2,1-*a*][2]benzazepin-13-yl)éthyl]acétamide, le *N*-[2-(11-méthoxy-6,7-dihydro-5*H*-pyrido[2',3':4,5]pyrrolo[2,1-*a*][2]benzazepin-13-yl) éthyl]-2-furamide, le *N*-[2-(11-hydroxy-6,7-dihydro-5*H*-pyrido[2',3':4,5]pyrrolo[2,1-*a*] [2]benzazepin-13-yl)éthyl]-2-furamide ainsi que leurs sels d'addition à un acide ou à une base pharmaceutiquement acceptable.

17. Composé de formule (I) selon la revendication 1 qui est le *N*-[2-(2-méthoxy-6-oxo-6*H*-pyrido[2',3':4,5]pyrrolo[2,1-*a*]isoindol-11-yl)éthyl]-2-furamide ainsi que ses sels d'addition à un acide ou à une base pharmaceutiquement acceptable.

18. Procédé de préparation des composés de formule (I) selon la revendication 1 **caractérisé en ce que** l'on utilise comme produit de départ le composé de formule (II) : 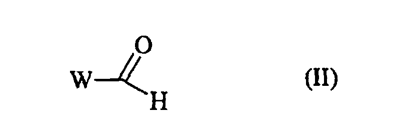 dans laquelle W représente un radical de formule (III) : 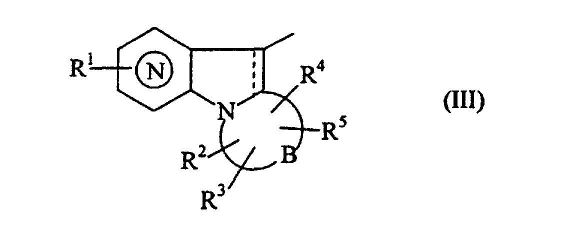 dans laquelle R¹, R², R³, R⁴, R⁵, B, la représentation 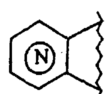 et la représentation ---- sont tels que définis précédemment,
que l'on soumet
- à un agent réducteur afin d'obtenir le composé de formule (IV) :
W-CH₂OH (IV)
dans laquelle W est tel que défini précédemment,
que l'on soumet après transformation en dérivé halogéné correspondant à l'action d'un sel de cyanure pour conduire au composé de formule (V) :
W-CH₂-CN (V)
dans laquelle W est tel que défini précédemment,
- ou successivement à une réaction de Wittig puis à une réduction pour obtenir le composé de formule (VI) :
W-G'₁-X (VI)
dans laquelle W est tel que défini précédemment , X représente un groupement CN ou COOalk (où alk représente un groupement alkyle) et G'₁ représente une chaîne telle que définie précédemment pour G₁ comportant 2 à 4 atomes de carbones,
composés de formules (V) et (VI) que l'on hydrolyse en milieu acide ou basique pour obtenir le composé de formule (VII) :
W-G₁-COOH (VII)
dans laquelle W et G₁ sont définis comme précédemment,
sur lequel on fait agir, en présence d'un agent de couplage, ou après transformation en chlorure d'acide correspondant, une amine HNRR' où R et R' sont définis comme précédemment pour conduire au composé de formule (I/a), cas particulier des composés de formule (I) : 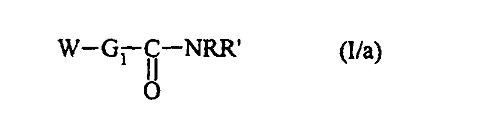 dans laquelle W, G₁, R et R' sont définis comme précédemment,
composé de formule (I/a) qui est soumis, dans le cas où R et R' représentent simultanément un atome d'hydrogène, à l'action de NaOBr afin de conduire, après hydrolyse, au composé de formule (VIII) :
W-G₁-NH₂ (VIII)
dans laquelle W et G₁ sont définis comme précédemment,
que l'on soumet à l'action :
- d'un chlorure d'acyle de formule (IX) : 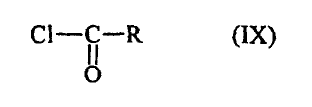 où R est tel que défini précédemment, ou l'anhydride d'acide (mixte ou symétrique) correspondant,
pour obtenir le composé de formule (I/b), cas particulier des composés de formule (I) : 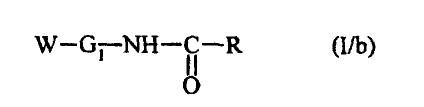 dans laquelle W, G₁ et R sont définis comme précédemment, composé de formule (I/b) pouvant par ailleurs être obtenu lorsque G₁ représente une chaîne (CH₂)₂, à partir du composé de formule (II) par action du nitrométhane pour conduire au composé de formule (III') : 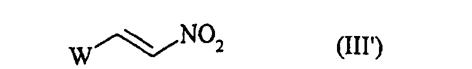 dans laquelle W est défini comme précédemment,
qui est soumis à l'action d'un agent réducteur comme NaBH₄, par exemple, pour obtenir le composé de formule (IV') 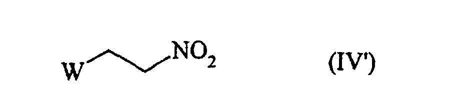 dans laquelle W est tel que défini précédemment,
que l'on soumet à une hydrogénation catalytique afin d'obtenir le composé de formule (V') : 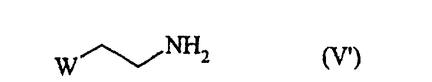 dans laquelle W est tel que défini précédemment,
sur lequel on fait agir un chlorure d'acide de formule (IX) ou l'anhydride d'acide (mixte ou symétrique) correspondant pour obtenir le composé de formule (I/b'), cas particulier des composés de formule (I/b) : 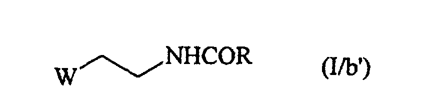 dans laquelle W et R sont définis comme précédemment,
- ou composé de formule (VIII) que l'on soumet à l'action d'un composé de formule (X)
O=C=N-R (X)
dans laquelle R est tel que défini précédemment,
pour conduire au composé de formule (I/c), cas particulier des composés de formule (I) : 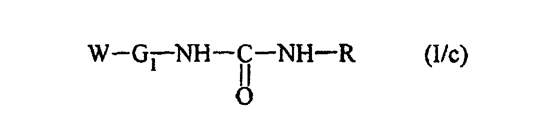 dans laquelle W, G₁ et R ont la même définition que précédemment,
les composés de formule (I/b) et (I/c) pouvant être soumis à l'action d'un composé de formule (XI) :
Rₐ-J (XI)
dans laquelle Rₐ peut prendre toutes les valeurs de R à l'exception de l'atome d'hydrogène et J représente un groupe partant comme un atome d'halogène ou un groupement tosyle,
pour conduire au composé de formule (I/d), cas particulier des composés de formule (I) : 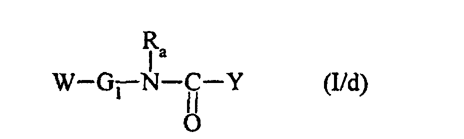 dans laquelle W, G₁ et Rₐ sont tels que définis précédemment et Y représente un groupement R ou -NRR' où R et R' sont tels que définis précédemment,
et/ou les composés de formules (I/a), (I/b), (I/c) et (I/d) peuvent être soumis à l'action d'un agent de thionation, comme le réactif de Lawesson pour conduire au composé de formule (I/e), cas particulier des composés de formule (I):
W-G₁-T (I/e)
dans laquelle W et G₁ sont définis de la même façon que précédemment et T représente un groupement 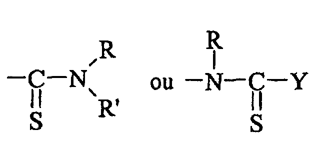 dans lesquels R, R' et Y sont tels que définis précédemment,
les composés (I/a) à (I/e) formant l'ensemble des composés de formule (I) et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

19. Composés de formule (XVII') : 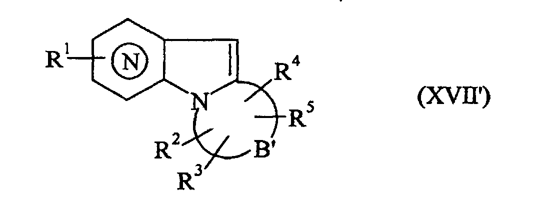 dans laquelle R¹, R², R³, R⁴, R⁵, et la représentation 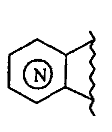 sont tels que définis dans la revendication 1 et B' représente un groupement B tel que défini dans la revendication 1 à l'exception du groupement pipérazinyle, utiles en tant qu'intermédiaires de synthèse des composés de formule (I).

20. Compositions pharmaceutiques contenant comme principe actif au moins un composé de formule (I) selon l'une quelconque des revendications 1 à 17 ou un de leurs sels d'addition avec un acide ou une base pharmaceutiquement acceptable en combinaison avec un ou plusieurs excipients pharmaceutiquement acceptables.

21. Compositions pharmaceutiques selon la revendication 20 utiles pour la fabrication d'un médicament pour traiter les troubles du système mélatoninergique.

## Patentansprüche

1. Verbindungen der Formel (I): 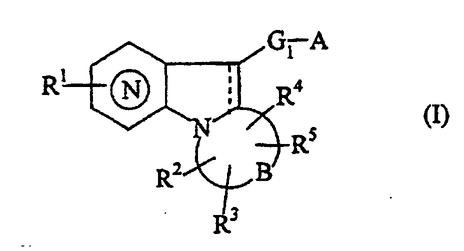 in der:
- das Symbol 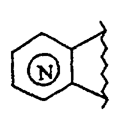 für die Gruppen 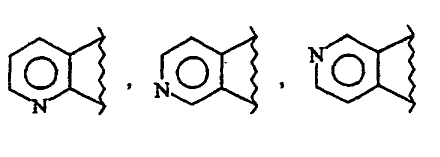 oder 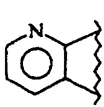 steht,
- R¹ ein Halogenatom oder eine Gruppe -R, -OR, -S(O)ₙR, -NRR', 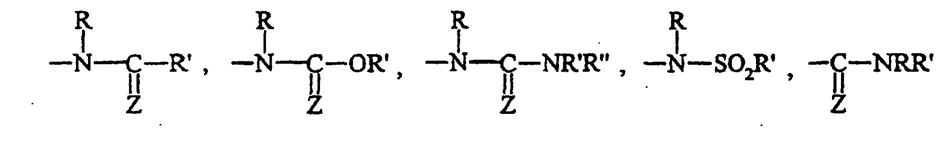 oder -SO₂NRR' darstellt (worin n 0, 1 oder 2, Z ein Schwefelatom oder Sauerstoffatom und R, R' und R", die gleichartig oder verschieden sind, Wasserstoffatome oder substituierte oder nichtsubstituierte, geradkettige oder verzweigte (C₁-C₆)-Alkylgruppen, substituierte oder nichtsubstituierte, geradkettige oder verzweigte (C₂-C₆)-Alkenylgruppen, substituierte oder nichtsubstituierte, geradkettige oder verzweigte (C₂-C₆)-Alkinylgruppen, substituierte oder nichtsubstituierte (C₃-C₈)-Cycloalkylgruppen, substituierte oder nichtsubstituierte, geradkettige oder verzweigte (C₃-C₈)-Cycloalkyl-(C₁-C₆)-alkylgruppen, Arylgruppen, geradkettige oder verzweigte Aryl-(C₁-C₆)-alkylgruppen, Heteroarylgruppen oder geradkettige oder verzweigte Heteroaryl-(C₁-C₆)-alkylgruppen darstellen, oder
(R und R') und (R' und R") weiterhin zusammen mit dem sie tragenden Stickstoffatom eine Morpholinyl-, Piperidinyl-, Piperazinyl- oder Pyrrolidinylgruppe bilden können, wobei diese Gruppen substituiert oder nichtsubstituiert sind),
- G₁ eine Alkylenkette mit 1 bis 4 Kohlenstoffatomen darstellt, die gegebenenfalls durch eine Gruppe R, OR, COR oder COOR (worin R die oben angegebenen Bedeutungen besitzt) substituiert ist,
oder G₁ eine Alkylenkette darstellt, die 1 bis 4 Kohlenstoffatome aufweist, in der eine der Gruppen CH₂ durch eine (C₃-C₈)-Cycloalkylengruppe ersetzt sein kann,
- A eine Gruppe 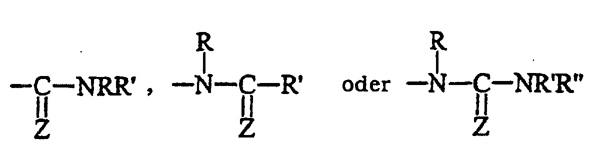 bedeutet, worin R, R', R" und Z die oben angegebenen Bedeutungen besitzen,
- B zusammen mit dem Stickstoffatom und dem Kohlenstoffatom, an das sie gebunden ist, einen Ring mit 5 bis 8 Kettengliedern bildet, der eine oder mehrere Unsättigungen aufweisen kann und neben dem Stickstoffatom ein zusätzliches Heteroatom ausgewählt aus Sauerstoff, Schwefel oder Stickstoff enthalten kann,
- R² und R³, die gleichartig oder verschieden sind, Wasserstoffatome oder geradkettige oder verzweigte (C₁-C₆)-Alkylgruppen, geradkettige oder verzweigte (C₁-C₆)-Alkoxygruppen oder Hydroxylgruppen bedeuten, oder R² und R³ gemeinsam eine Oxogruppe bilden,
- R⁴ und R⁵ Wasserstoffatome bedeuten oder gemeinsam mit zwei benachbarten sie tragenden Atomen des Ringes B eine Gruppe ausgewählt aus Aryl oder Heteroaryl darstellen.
- das Symbol ----- bedeutet, daß die Bindung einfach oder doppelt ist, wobei es sich versteht, daß die Wertigkeit der Atome berücksichtigt wind,
mit der Maßgabe, daß:
- der Begriff «substituiert», bezogen auf die Begriffe «Alkyl», «Alkenyl», «Alkinyl», «Cycloalkyl», «Morpholinyl», «Piperidinyl», «Piperazinyl» und «Pyrrolidinyl», bedeutet, daß diese Gruppen durch eine oder mehrere Gruppen ausgewählt aus Hydroxy, Oxo, Alkoxy, Alkyl, Polyhalogenalkyl oder Halogenatomen substituiert sein können,
- der Begriff «substituiert», bezogen auf den Begriff «Cycloalkylalkyl» bedeutet, daß der cyclische Teil der Gruppe durch eine oder mehrere Gruppen ausgewählt aus Hydroxy, Oxo, Alkoxy, Alkyl, Polyhalogenalkyl oder Halogenatomen substituiert sein kann,
- unter «Aryl» man eine Phenyl-, Naphthyl- oder Biphenylgruppe versteht, wobei diese Gruppen unsubstituiert sind oder substituiert durch eine oder mehrere, gleichartige oder verschiedene Gruppen ausgewählt aus Hydroxy, Alkoxy, Alkyl, Amino, Alkylamino, Dialkylamino, Nitro, Cyano, Polyhalogenalkyl, Formyl, Carboxy, Alkoxycarbonyl, Amido oder Halogenatomen,
- unter «Heteroaryl» man eine Furyl-, Pyrrolyl-, Imidazolyl-, Pyridyl-, Thienyl-, Pyrazinyl-, Benzothienyl-, Benzofuranyl-, Indolyl-, Benzimidazolyl-, Chinolyloder Chinazolinylgruppe versteht, wobei diese Gruppen nichtsubstituiert sind oder substituiert durch eine oder mehrere, gleichartige oder verschiedene Gruppen ausgewählt aus Hydroxy, Alkoxy, Alkyl, Amino, Alkylamino, Dialkylamino, Nitro, Cyano, Polyhalogenalkyl, Formyl, Carboxy, Amido und Halogenatomen,
deren Enantiomere und Diastereoisomere sowie deren Additionssalze mit einer pharmazeutisch annehmbaren Säure oder Base.

2. Verbindungen der Formel (I) nach Anspruch 1: 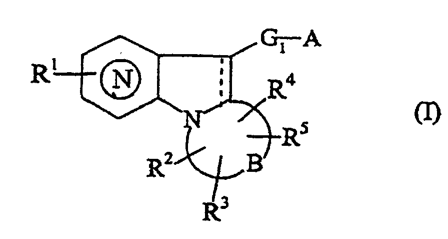 in der:
- das Symbol 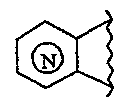 für die Gruppen 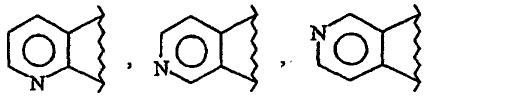 oder 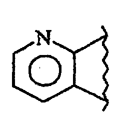 steht,
- R¹ ein Halogenatom oder eine Gruppe -R, -OR, -S(O)ₙR, -NRR', 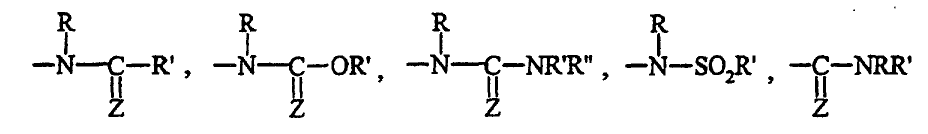 oder -SO₂NRR' bedeutet (worin n 0, 1 oder 2, Z ein Schwefelatom oder ein Sauerstoffatom und R, R' und R", die gleichartig oder verschieden sind, Wasserstoffatome oder geradkettige oder verzweigte, substituierte oder nichtsubstituierte (C₁-C₆)-Alkylgruppen, geradkettige oder verzweigte, substituierte oder nichtsubstituierte (C₂-C₆)-Alkenylgruppen, geradkettige oder verzweigte, substituierte oder nichtsubstituierte (C₂-C₆)-Alkinylgruppen, suhstituierte oder nichtsubstituierte (C₃-C₈)-Cycloalkylgruppen, substituierte oder nichtsubstituierte (C₃-C₈)-Cycloalkyl-(C₁-C₆)-alkylgruppen, Arylgruppen, geradkettige oder verzweigte Aryl-(C₁-C₆)-alkylgruppen, Heteroarylgruppen oder geradkettige oder verzweigte Heteroaryl-(C₁-C₆)-alkylgruppen darstellen; und
(R und R') und (R' und R") zusätzlich zusammen mit dem sie tragenden Stickstoffatom eine Morpholinyl-, Piperidinyl-, Piperazinyl- oder Pyrrolidinylgruppe bilden können, wobei diese Gruppen nichtsubstituiert oder substituiert sind),
- G₁ eine Alkylenkette darstellt, die 1 bis 4 Kohlenstoffatome aufweist und gegebenenfalls durch eine Gruppe R, OR, COR oder COOR substituiert ist (worin R die oben angegebenen Bedeutungen besitzt),
- A eine Gruppe 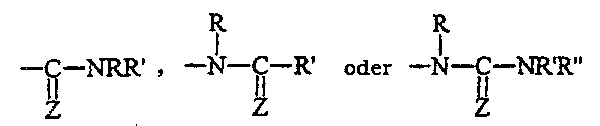 bedeutet, worin R, R', R" und Z die oben angegebenen Bedeutungen besitzen,
- B zusammen mit dem Stickstoffatom und dem Kohlenstoffatom, an die sie gebunden ist, einen Ring mit 5 bis 8 Kettengliedern bildet, der eine oder mehrere Unsättigungen und neben dem Stickstoffatom ein zusätzliches Heteroatom ausgewählt aus Sauerstoff, Schwefel oder Stickstoff enthalten kann,
- R² und R³, die gleichartig oder verschieden sind, Wasserstoffatome oder geradkettige oder verzweigte (C₁-C₆)-Alkylgruppen, geradkettige oder verzweigte (C₁-C₆)-Alkoxygruppen oder Hydroxylgruppen bedeuten, oder R² und R³ gemeinsam eine Oxogruppe bilden,
- R⁴ und R⁵ Wasserstoffatome bedeuten oder zusammen mit den beiden sie tragenden Atomen des Ringes B eine Gruppe ausgewählt aus Aryl oder Heteroaryl bilden,
- das Symbol ----- bedeutet, daß die Bindung einfach oder doppelt ist, wobei es sich versteht, daß die Wertigkeit der Atome berücksichtigt wird,
mit der Maßgabe, daß:
- der Begriff «substituiert», bezogen auf die Begriffe «Alkyl», «Alkenyl», «Alkinyl», «Cycloalkyl», «Morpholinyl», «Piperidinyl», «Piperazinyl» und «Pyrrolidinyl», bedeutet, daß diese Gruppen durch eine oder mehrere Gruppen ausgewählt aus Hydroxy, Oxo, Alkoxy, Alkyl, Polyhalogenalkyl oder Halogenatomen substituiert sein können,
- der Begriff «substituiert», bezogen auf den Begriff «Cycloalkylalkyl» bedeutet, daß der cyclische Teil der Gruppe durch eine oder mehrere Gruppen ausgewählt aus Hydroxy, Oxo, Alkoxy, Alkyl, Polyhalogenalkyl oder Halogenatomen substituiert sein kann,
- unter «Aryl» man eine Phenyl-, Naphthyl- oder Biphenylgruppe versteht, wobei diese Gruppen unsubstituiert sind oder substituiert durch eine oder mehrere, gleichartige oder verschiedene Gruppen ausgewählt aus Hydroxy, Alkoxy, Alkyl, Amino, Alkylamino, Dialkylamino, Nitro, Cyano, Polyhalogenalkyl, Formyl, Carboxy, Alkoxycarbonyl, Amido oder Halogenatomen,
- unter «Heteroaryl» man eine Furyl-, Pyrrolyl-, Imidazolyl-, Pyridyl-, Thienyl-, Pyrazinyl-, Benzothienyl-, Benzofuranyl-, Indolyl-, Benzimidazolyl-, Chinolyloder Chinazolinylgruppe versteht, wobei diese Gruppen nichtsubstituiert sind oder substituiert durch eine oder mehrere, gleichartige oder verschiedene Gruppen ausgewählt aus Hydroxy, Alkoxy, Alkyl, Amino, Alkylamino, Dialkylamino, Nitro, Cyano, Polyhalogenalkyl, Formyl, Carboxy, Amido oder Halogenatomen,
deren Enantiomere und Diastereoisomere sowie deren Additionssalze mit einer pharmazeutisch annehmbaren Säure oder Base.

3. Verbindungen der Formel (I) nach Anspruch 1: 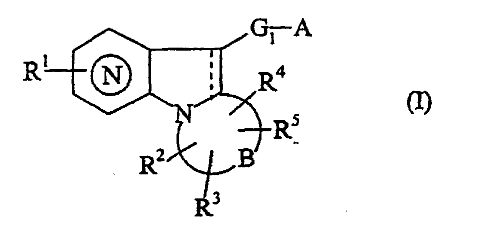 in der:
- das Symbol 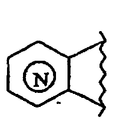 für die Gruppen 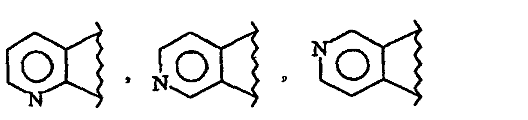 oder 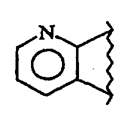 steht,
- R¹ ein Halogenatom oder eine Gruppe -R, -OR, -S(O)ₙR, -NRR', 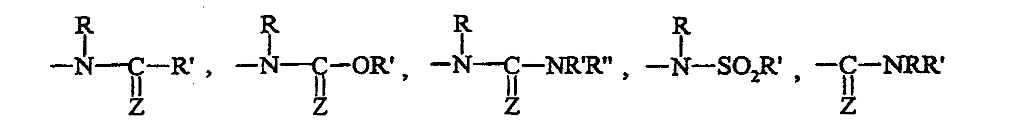 oder -SO₂NRR' darstellt (worin n 0, 1 oder 2, Z ein Schwefelatom oder Sauerstoffatom und R, R' und R", die gleichartig oder verschieden sind, Wasserstoffatome oder geradkettige oder verzweigte, substituierte oder nichtsubstituierte (C₁-C₆)-Alkylgruppen, geradkettige oder verzweigte, substituierte oder nichtsubstituierte (C₂-C₆)-Alkenylgruppen, geradkettige oder verzweigte, substituierte oder nichtsubstituierte (C₂-C₆)-Alkinylgruppen, substituierte oder nichtsubstituierte (C₃-C₈)-Cycloalkylgruppen, geradkettige oder verzweigte, substituierte oder nichtsubstituierte (C₃-C₈)-Cycloalkyl-(C₁-C₆)-alkylgruppen, Arylgruppen, geradkettige oder verzweigte Aryl-(C₁-C₆)-alkylgruppen, Heteroarylgruppen oder geradkettige oder verzweigte Heteroaryl-(C₁-C₆)-alkylgruppen darstellen, oder
(R und R') und (R' und R") weiterhin zusammen mit dem sie tragenden Stickstoffatom eine Morpholinyl-, Piperidinyl-, Piperazinyl- oder Pyrrolidinylgruppe bilden können, wobei diese Gruppen substituiert oder nichtsubstituiert sind),
- G₁ eine Alkylenkette mit 1 bis 4 Kohlenstoffatomen darstellt, in der eine der Gruppen CH₂ durch eine (C₃-C₈)-Cycloalkylengruppe ersetzt sein kann,
- A eine Gruppe 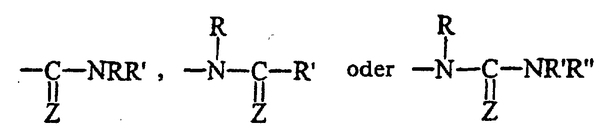 bedeutet, worin R, R', R" und Z die oben angegebenen Bedeutungen besitzen,
- B zusammen mit dem Stickstoffatom und dem Kohlenstoffatom, an das sie gebunden ist, einen Ring mit 5 bis 8 Kettengliedern bildet, der eine oder mehrere Unsättigungen aufweisen kann und neben dem Stickstoffatom ein zusätzliches Heteroatom ausgewählt aus Sauerstoff, Schwefel oder Stickstoff enthalten kann,
- R² und R³, die gleichartig oder verschieden sind, Wasserstoffatome oder geradkettige oder verzweigte (C₁-C₆)-Alkylgruppen, geradkettige oder verzweigte (C₁-C₆)-Alkoxygruppen oder Hydroxylgruppen bedeuten,
oder R² und R³ gemeinsam eine Oxogruppe bilden,
- R⁴ und R⁵ Wasserstoffatome bedeuten oder gemeinsam mit zwei benachbarten sie tragenden Atomen des Ringes B eine Gruppe ausgewählt aus Aryl oder Heteroaryl darstellen,
- das Symbol ----- bedeutet, daß die Bindung einfach oder doppelt ist, wobei es sich versteht, daß die Wertigkeit der Atome berücksichtigt wird,
mit der Maßgabe, daß:
- der Begriff «substituiert», bezogen auf die Begriffe «Alkyl», «Alkenyl», «Alkinyl», «Cycloalkyl», «Morpholinyl», «Piperidinyl», «Piperazinyl» und «Pyrrolidinyl», bedeutet, daß diese Gruppen durch eine oder mehrere Gruppen ausgewählt aus Hydroxy, Oxo, Alkoxy, Alkyl, Polyhalogenalkyl oder Halogenatomen substituiert sein können,
- der Begriff «substituiert», bezogen auf den Begriff «Cycloalkylalkyl» bedeutet, daß der cyclische Teil der Gruppe durch eine oder mehrere Gruppen ausgewählt aus Hydroxy, Oxo, Alkoxy, Alkyl, Polyhalogenalkyl oder Halogenatomen substituiert sein kann,
- unter «Aryl» man eine Phenyl-, Naphthyl- oder Biphenylgruppe versteht, wobei diese Gruppen unsubstituiert sind oder substituiert durch eine oder mehrere, gleichartige oder verschiedene Gruppen ausgewählt aus Hydroxy, Alkoxy, Alkyl, Amino, Alkylamino, Dialkylamino, Nitro, Cyano, Polyhalogenalkyl, Formyl, Carboxy, Alkoxycarbonyl, Amido oder Halogenatomen,
- unter «Heteroaryl» man eine Furyl-, Pyrrolyl-, Imidazolyl-, Pyridyl-, Thienyl-, Pyrazinyl-, Benzothienyl-, Benzofuranyl-, Indolyl-, Benzimidazolyl-, Chinolyloder Chinazolinylgruppe versteht, wobei diese Gruppen nichtsuhstituiert sind oder substituiert durch eine oder mehrere, gleichartige oder verschiedene Gruppen ausgewählt aus Hydroxy, Alkoxy, Alkyl, Amino, Alkylamino, Dialkylamino, Nitro, Cyano, Polyhalogenalkyl, Formyl, Carboxy, Amido und Halogenatomen,
deren Enantiomere und Diastereoisomere sowie deren Additionssalze mit einer pharmazeutisch annehmbaren Säure oder Base.

4. Verbindungen der Formel (I) nach Anspruch 1, worin B zusammen mit dem Stickstoffatom und dem Kohlenstoffatom, an die es gebunden ist, einen Pyrrolidin-, Piperidin-, (Perhydro)azepin- oder (Perhydro)azocin-Ring bildet, deren Enantiomere und Diastereoisomere sowie deren Additionssalze mit einer pharmazeutisch annehmbaren Säure oder Base.

5. Verbindungen der Formel (I) nach Anspruch 1, worin B zusammen mit dem Stickstoffatom und dem Kohlenstoffatom, an die es gebunden ist, einen 1,3-(Perhydro)oxazin-Ring bildet, deren Enantiomere und Diastereoisomere sowie deren Additionssalze mit einer pharmazeutisch annehmbaren Säure oder Base.

6. Verbindungen der Formel (I) nach Anspruch 1, worin R², R³, R⁴ und R⁵ gleichzeitig Wasserstoffatome bedeuten, deren Enantiomere und Diastereoisomere sowie deren Additionssalze mit einer pharmazeutisch annehmbaren Säure oder Base.

7. Verbindungen der Formel (I) nach Anspruch 1, worin R² und R³ Wasserstoffatome bedeuten und R⁴ und R⁵ gemeinsam mit sie tragenden benachbarten Kohlenstoffatomen eine Phenylgruppe bilden, deren Enantiomere und Diastereoisomere sowie deren Additionssalze mit einer pharmazeutisch annehmbaren Säure oder Base.

8. Verbindungen der Formel (I) nach Anspruch 1, worin R¹ eine Gruppe OR darstellt, deren Enantiomere und Diastereoisomere sowie deren Additionssalze mit einer pharmazeutisch annehmbaren Säure oder Base.

9. Verbindungen der Formel (I) nach Anspruch 1, worin G₁ eine Gruppe (CH₂)ₚ darstellt, worin p den Wert 2 oder 3 besitzt, deren Enantiomere und Diastereoisomere sowie deren Additionssalze mit einer pharmazeutisch annehmbaren Säure oder Base.

10. Verbindungen der Formel (I) nach Anspruch 1, worin A eine Gruppe NHCOR darstellt, deren Enantiomere und Diastereoisomere sowie deren Additionssalze mit einer pharmazeutisch annehmbaren Säure oder Base.

11. Verbindungen der Formel (I) nach Anspruch 1, worin A eine Gruppe CONHR darstellt, deren Enantiomere und Diastereoisomere sowie deren Additionssalze mit einer pharmazeutisch annehmbaren Säure oder Base.

12. Verbindungen der Formel (I) nach Anspruch 1, nämlich *N*-[2-(2-Methoxy-6*H*-pyrido[2',3':4,5]pyrrolo[2,1-*a*]isoindol-11-yl)-ethyl]-acetamid, *N*-[2-(2-Methoxy-6*H*-pyrido[2',3':4,5]pyrrolo[2,1-*a*]isoindol-11-yl)-ethyl]-2-furancarbonsäureamid, N-[2-(2-Methoxy-6*H*-pyrido[2',3':4,5]pyrrolo[2,1-*a*]isoindol-11-yl)-ethyl]-benzamid, 2-Iod-*N*-[2-(2-methoxy-6*H*-pyrido[2',3':4,5]pyrrolo[2,1-*a*]isoindol-11-yl)-ethyl]-benzamid sowie deren Additionssalze mit einer pharmazeutisch annehmbaren Säure oder Base.

13. Verbindungen der Formel (I) nach Anspruch 1, nämlich *N*-[2-(3-Methoxy-6,7,8,9-tetrahydropyrido[3,2-*b*]indolizin-5-yl)-ethyl]-acetamid, *N*-[2-(2-Methoxy-6,7,8,9-tetrahydropyrido[2,3-*b*]indolizin-10-yl)-ethyl]-2-furancarbonsäureamid, *N*-[2-(2-Methoxy-6,7,8,9-tetrahydropyrido[2,3-*b*]indolizin-10-yl)-ethyl]-acetamid, *N*-[2-(2-Hydroxy-6,7,8,9-tetrahydropyrido[2,3-*b*]-indolizin-10-yl)-ethyl]-2-furancarbonsäureamid sowie deren Additionssalze mit einer pharmazeutisch annehmbaren Säure oder Base.

14. Verbindungen der Formel (I) nach Anspruch 1, nämlich *N*-[2-(8-Methoxy-3,4-dihydro-2*H*-pyrido[2',3':4,5]pyrrolo[2,1-*b*][1,3]oxazin-10-yl)-ethyl]-acetamid, *N*-[2-(8-Methoxy-3,4-dihydro-2H-pyrido[2',3':4,5]pyrrolo[2,1-*b*][1,3]oxazin-10-yl)-ethyl]-2-furancarbonsäureamid sowie deren Additionssalze mit einer pharmazeutisch annehmbaren Säure oder Base.

15. Verbindung der Formel (I) nach Anspruch 1, nämlich *N*-(2-(10-Methoxy-5,6-dihydro-pyrido[2',3':4,5]pyrrolo[2,1-*a*]isochinolin-12-yl)-ethyl]-2-furancarbonsäureamid sowie dessen Additionssalze mit einer pharmazeutisch annehmbaren Säure oder Base.

16. Verbindungen der Formel (I) nach Anspruch 1, nämlich *N*-[2-(11-Methoxy-6,7-dihydro-5*H*-pyrido[2',3':4,5]pyrrolo[2,1-*a*][2]benzazepin-13-yl)-ethyl]-acetamid, *N*-[2-(11-Methoxy-6,7-dihydro-5H-pyrido[2',3':4,5]pyrrolo[2,1-*a*][2]benzazepin-13-yl)-ethyl]-2-furancarbonsäureamid, *N*-[2-(11-Hydroxy-6,7-dihydro-5*H*-pyrido-[2',3':4,5]pyrrolo[2,1-*a*][2]benzazepin-13-yl)-ethyl]-2-furancarbonsäureamid sowie deren Additionssalze mit einer pharmazeutisch annehmbaren Säure oder Base.

17. Verbindung der Formel (I) nach Anspruch 1, nämlich *N*-[2-(2-Methoxy-6-oxo-6H-pyrido[2',3':4,5]pyrrolo[2,1-*a*]isoindol-11-yl)-ethyl]-2-furancarbonsäureamid sowie dessen Additionssalze mit einer pharmazeutisch annehmbaren Säure oder Base.

18. Verfahren zur Herstellung der Verbindungen der Formel (I) nach Anspruch 1, **dadurch gekennzeichnet, daß** man als Ausgangsprodukt die Verbindung der Formel (II) verwendet: 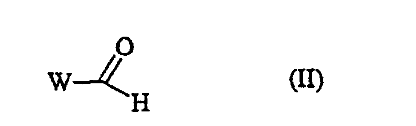 in der W eine Gruppe der Formel (III) darstellt: 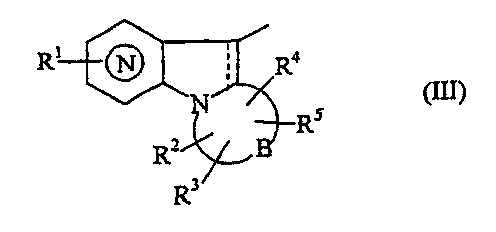 worin R¹, R², R³, R⁴, R⁵, B und das Symbol 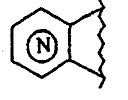 und das Symbol ---- die oben angegebenen Bedeutungen besitzen,
welche man
- der Einwirkung eines Reduktionsmittels unterwirft zur Bildung der Verbindung der Formel (IV):
W - CH₂OH (IV)
in der W die oben angegebenen Bedeutungen besitzt,
welche man nach der Umwandlung in das entsprechende Halogen der Einwirkung eines Cyanidsalzes unterwirft zur Bildung der Verbindung der Formel (V):
W - CH₂ - CN (V)
in der W die oben angegebenen Bedeutungen besitzt,
- oder nacheinander einer Wittig-Reaktion und dann einer Reduktion unterwirft zur Bildung der Verbindung der Formel (VI):
W - G'₁ - X (VI)
in der W die oben angegebenen Bedeutungen besitzt, X eine Gruppe CN oder COOAlk (worin Alk für eine Alkylgruppe steht) und G'₁ eine Kette, wie sie oben für G₁ definiert worden ist, die 2 bis 4 Kohlenstoffatome aufweist, bedeuten,
welche Verbindungen der Formeln (V) und (VI) man in saurem oder basischem Medium hydrolysiert zur Bildung der Verbindung der Formel (VII):
W - G₁ - COOH (VII)
in der W und G₁ die oben angegebenen Bedeutungen besitzen,
welche man in Gegenwart eines Kupplungsmittels oder nach der Umwandlung in das entsprechende Säurechlorid mit einem Amin HNRR', worin Rund R' die oben angegebenen Bedeutungen besitzen, umsetzt zur Bildung de Verbindung der Formel (I/a), einem Sonderfall der Verbindungen der Formel (I): 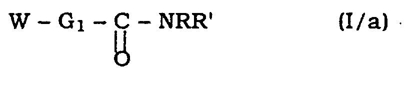 in der W, G₁, R und R' die oben angegebenen Bedeutungen besitzen,
welche Verbindung der Formel (I/a) dann, wenn R und R' gleichzeitig Wasserstoffatome bedeuten, der Einwirkung von NaOBr unterworfen wird, so daß man nach der Hydrolyse die Verbindung der Formel (VIII) erhält:
W - G₁ - NH₂ (VIII)
in der W und G₁ die oben angegebenen Bedeutungen besitzen,
welche man der Einwirkung:
- eines Acylchlorids der Formel (IX): 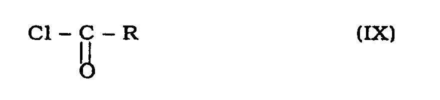 worin R die oben angegebenen Bedeutungen besitzt, oder des entsprechenden (gemischten oder symmetrischen) Säureanhydrids unterwirft
zur Bildung der Verbindung der Formel (I/b), einem Sonderfall der Verbindungen der Formel (I): 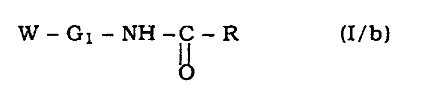 in der W, G₁ und R die oben angegebenen Bedeutungen besitzen,
welche Verbindung der Formel (I/b), wenn G₁ eine Kette (CH₂)₂ bedeutet, erhalten werden kann ausgehend von der Verbindung der Formel (II) durch Einwirkung von Nitromethan zur Bildung der Verbindung der Formel (III'): 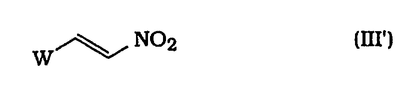 in der W die oben angegebenen Bedeutungen besitzt,
welche der Einwirkung eines Reduktionsmittels, wie beispielsweise NaBH₄, unterworfen wird zur Bildung der Verbindung der Formel (IV'): 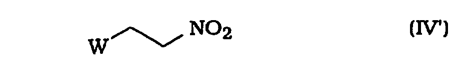 in der W die oben angegebenen Bedeutungen besitzt,
welche man einer katalytischen Hydrierung unterwirft, so daß man schließlich die Verbindung der Formel (V') erhält: 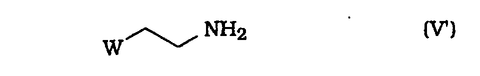 in der W die oben angegebenen Bedeutungen besitzt,
auf welche man ein Säurechlorid der Formel (IX) oder das entsprechende (gemischte oder symmetrische) Säureanhydrid einwirken läßt zur Bildung der Verbindung der Formel (I/b'), einem Sonderfall der Verbindungen der Formel (I/b): 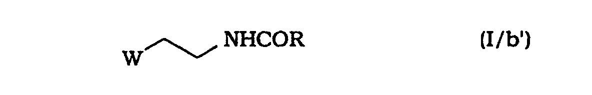 in der W und R die oben angegebenen Bedeutungen besitzen,
- oder man die Verbindung der Formel (VIII) der Einwirkung einer Verbindung der Formel (X):
O = C = N - R (X)
in der R die oben angegebenen Bedeutungen besitzt,
unterwirft zur Bildung der Verbindung der Formel (I/c), einem Sonderfall der Verbindungen der Formel (I): 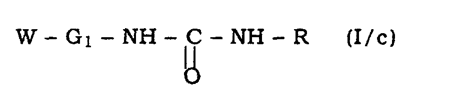 in der W, G₁ und R die oben angegebenen Bedeutungen besitzen,
welche Verbindungen der Formeln (I/b) und (I/c) der Einwirkung einer Verbindung der Formel (XI):
Rₐ - J (XI)
in der Rₐ sämtliche Bedeutungen von R mit Ausnahme des Wasserstoffatoms besitzen kann und J eine austretende Gruppe bedeutet, wie ein Halogenatom oder eine Tosylgruppe, unterworfen werden kann
zur Bildung der Verbindung der Formel (I/d), einem Sonderfall der Verbindungen der Formel (I): 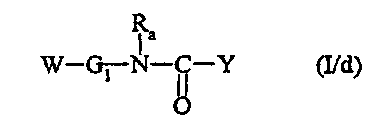 in der W, G₁ und Rₐ die oben angegebenen Bedeutungen besitzen und Y eine Gruppe R odr -NRR' darstellt, worin R und R' die oben angegebenen Bedeutungen besitzen.
und/oder die Verbindungen der Formeln (I/a), (I/b), (I/c) und (I/d) der Einwirkung eines Thionierungsmittels, wie des Lawesson-Reagens unterworfen werden können zur Bildung der Verbindung der Formel (I/e), einem Sonderfall der Verbindungen der Formel (I):
W - G₁ - T (I/e)
in der W und G₁ die oben angegebenen Bedeutungen besitzen und T eine Gruppe 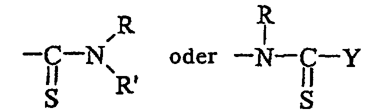 darstellt, worin R, R' und Y die oben angegebenen Bedeutungen besitzen,
wobei die Verbindungen (I/a) bis (I/e) die Gesamtheit der Verbindungen der Formel (I) bilden und die mit Hilfe einer klassischen Reinigungsmethode gereinigt werden können und die man gewünschtenfalls mit einer pharmazeutisch annehmbaren Säure oder Base in ihre Additionssalze überführen kann und die man gegebenenfalls mit Hilfe einer klassischen Trennmethode in ihre Isomeren trennen kann.

19. Verbindungen der Formel (XVII'): 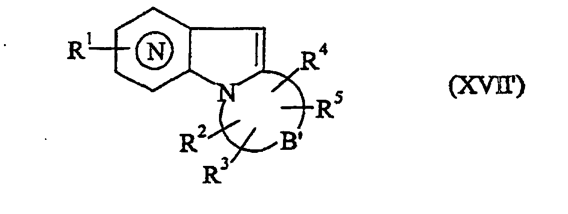 in der R¹, R², R³, R⁴, R⁵ und das Symbol 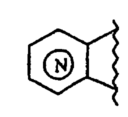 die in Anspruch 1 angegebenen Bedeutungen besitzen und B' eine Gruppe B darstellt, wie sie in Anspruch 1 definiert worden ist, mit Ausnahme der Piperazinylgruppe, als Zwischenproduke für die Synthese der Verbindungen der Formel (I).

20. Pharmazeutische Zubereitungen enthaltend als Wirkstoff mindestens eine Verbindung der Formel (I) nach einem der Ansprüche 1 bis 17 oder eines ihrer Additionssalze mit einer pharmazeutisch annehmbaren Säure oder Base in Kombination mit einem oder mehreren pharmazeutisch annehmbaren Trägermaterialien.

21. Pharmazeutische Zubereitungen nach Anspruch 20 für die Herstellung eines Arzneimittels zur Behandlung von Störungen des melatoninergischen Systems.

## Claims

1. Compounds of formula (I) : 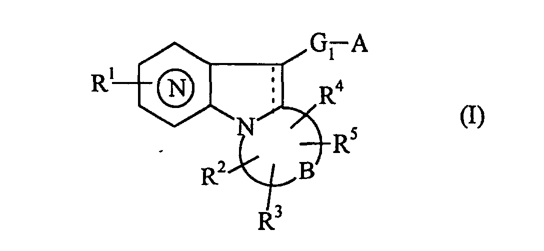 wherein :
- the symbo 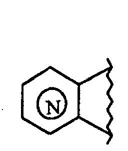 represents the grouping 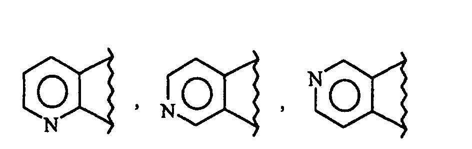 or 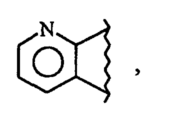
- R¹ represents a halogen atom or a group -R, -OR, -S(O)ₙR, -NRR', 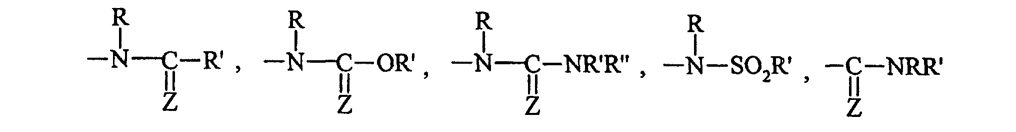 or -SO₂NRR' (wherein n is 0, 1 or 2, Z represents a sulphur or oxygen atom and R, R' and R", which may be identical or different, represent a hydrogen atom or an unsubstituted or substituted linear or branched (C₁-C₆)alkyl group, an unsubstituted or substituted linear or branched (C₂-C₆)alkenyl group, an unsubstituted or substituted linear or branched (C₂-C₆)alkynyl group, an unsubstituted or substituted (C₃-C₈)cycloalkyl group, an unsubstituted or substituted (C₃-C₈)cycloalkyl-(C₁-C₆)alkyl group in which the alkyl moiety is linear or branched, an aryl group, an aryl-(C₁-C₆)alkyl group in which the alkyl moiety is linear or branched, a heteroaryl group or a heteroaryl-(C₁-C₆)alkyl group in which the alkyl moiety is linear or branched,
it also being possible for (R and R') and (R' and R") to form together with the nitrogen atom carrying them a morpholinyl, piperidinyl, piperazinyl or pyrrolidinyl group, those groups being unsubstituted or substituted,
- G₁ represents an alkylene chain having from I to 4 carbon atoms, optionally substituted by a group R, OR, COR or COOR (wherein R is as defined hereinbefore), or G₁ represents an alkylene chain having from 1 to 4 carbon atoms wherein one of the CH₂ groups may be replaced by a (C₃-C₈)cycloalkylene group,
- A represents a group 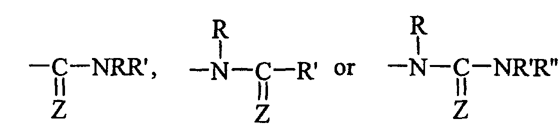
wherein R, R', R" and Z are as defined hereinbefore,
- B forms with the nitrogen atom and the carbon atom to which it is attached a ring having from 5 to 8 ring members, which may contain one or more unsaturated bonds and may contain, in addition to the nitrogen atom, an additional hetero atom selected from oxygen, sulphur and nitrogen,
- R² and R³, which may be identical or different, represent a hydrogen atom or a linear or branched (C₁-C₆)alkyl group, a linear or branched (C₁-C₆)alkoxy group or a hydroxy group,
or R² and R³ together form an oxo group,
- R⁴ and R⁵ represent a hydrogen atom or form together with two adjacent atoms of the B ring carrying them a group selected from aryl and heteroaryl,
- the symbol ----- means that the bond may be single or double, with the proviso that the valence of the atoms is respected,
it being understood that :
- the term "substituted" applied to the terms "alkyl", "alkenyl", "alkynyl", "cycloalkyl", "morpholinyl", "piperidinyl", "piperazinyl" and "pyrrolidinyl" means that those groups may be substituted by one or more groups selected from hydroxy, oxo, alkoxy, alkyl, polyhaloalkyl, and halogen atoms,
- the term "substituted" applied to the term "cycloalkylalkyl" means that the cyclic moiety of the group is substituted by one or more groups selected from hydroxy, oxo, alkoxy, alkyl, polyhaloalkyl, and halogen atoms,
- "aryl" is understood to mean a phenyl, naphthyl or biphenyl group, those groups being unsubstituted or substituted by one or more identical or different groups selected from hydroxy, alkoxy, alkyl, amino, alkylamino, dialkylamino, nitro, cyano, polyhaloalkyl, formyl, carboxy, alkoxycarbonyl, amido, and halogen atoms,
- "heteroaryl" is understood to mean a furyl, pyrrolyl, imidazolyl, pyridyl, thienyl, pyrazinyl, benzothienyl, benzofuranyl, indolyl, benzimidazolyl, quinolyl or quinazolinyl group, those groups being unsubstituted or substituted by one or more identical or different groups selected from hydroxy, alkoxy, alkyl, amino, alkylamino, dialkylamino, nitro, cyano, polyhaloalkyl, formyl, carboxy, amido, and halogen atoms,
their enantiomers and diastereoisomers, and addition salts thereof with a pharmaceutically acceptable acid or base.

2. Compounds of formula (I) according to claim 1 : 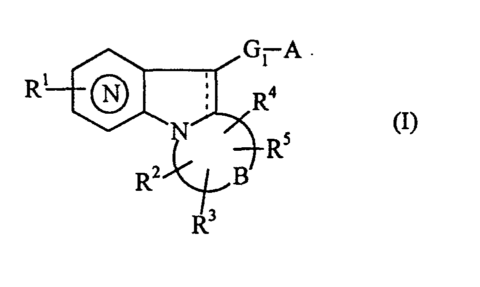 wherein :
- the symbol 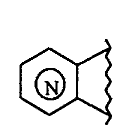 represents the grouping 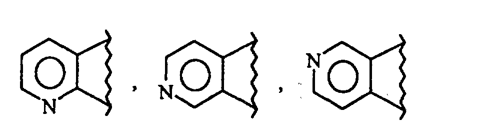 or 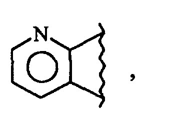
- R¹ represents a halogen atom or a group -R, -OR, -S(O)ₙR, -NRR', 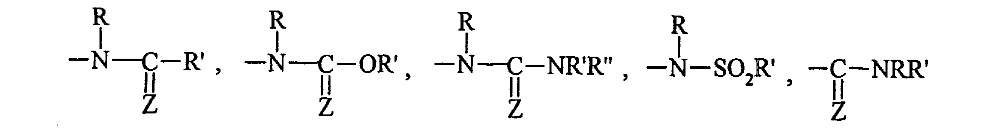 or -SO₂NRR' (wherein n is 0, 1 or 2, Z represents a sulphur or oxygen atom and R, R' and R", which may be identical or different, represent a hydrogen atom or an unsubstituted or substituted linear or branched (C₁-C₆)alkyl group, an unsubstituted or substituted linear or branched (C₂-C₆)alkenyl group, an unsubstituted or substituted linear or branched (C₂-C₆)alkynyl group, an unsubstituted or substituted (C₃-C₈)cycloalkyl group, an unsubstituted or substituted (C₃-C₈)cycloalkyl-(C₁-C₆)alkyl group in which the alkyl moiety is linear or branched, an aryl group, an aryl-(C₁-C₆)alkyl group in which the alkyl moiety is linear or branched, a heteroaryl group or a heteroaryl-(C₁-C₆)alkyl group in which the alkyl moiety is linear or branched,
it also being possible for (R and R') and (R' and R") to form together with the nitrogen atom carrying them a morpholinyl, piperidinyl, piperazinyl or pyrrolidinyl group, those groups being unsubstituted or substituted,
- G₁ represents an alkylene chain having from 1 to 4 carbon atoms, optionally substituted by a group R, OR, COR or COOR (wherein R is as defined hereinbefore),
- A represents a group 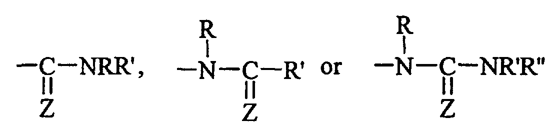
wherein R, R', R" and Z are as defined hereinbefore,
- B forms with the nitrogen atom and the carbon atom to which it is attached a ring having from 5 to 8 ring members, which may contain one or more unsaturated bonds and may contain, in addition to the nitrogen atom, an additional hetero atom selected from oxygen, sulphur and nitrogen,
- R² and R³, which may be identical or different, represent a hydrogen atom or a linear or branched (C₁-C₆)alkyl group, a linear or branched (C₁-C₆)alkoxy group or a hydroxy group,
or R² and R³ together form an oxo group,
- R⁴ and R⁵ represent a hydrogen atom or form together with two adjacent atoms of the B ring carrying them a group selected from aryl and heteroaryl,
- the symbol ----- means that the bond may be single or double, with the proviso that the valence of the atoms is respected,
it being understood that :
- the term "substituted" applied to the terms "alkyl", "alkenyl", "alkynyl", "cycloalkyl", "morpholinyl", "piperidinyl", "piperazinyl" and "pyrrolidinyl" means that those groups may be substituted by one or more groups selected from hydroxy, oxo, alkoxy, alkyl, polyhaloalkyl, and halogen atoms,
- the term "substituted" applied to the term "cycloalkylalkyl" means that the cyclic moiety of the group is substituted by one or more groups selected from hydroxy, oxo, alkoxy, alkyl, polyhaloalkyl, and halogen atoms,
- "aryl" is understood to mean a phenyl, naphthyl or biphenyl group, those groups being unsubstituted or substituted by one or more identical or different groups selected from hydroxy, alkoxy, alkyl, amino, alkylamino, dialkylamino, nitro, cyano, polyhaloalkyl, formyl, carboxy, alkoxycarbonyl, amido, and halogen atoms,
- "heteroaryl" is understood to mean a furyl, pyrrolyl, imidazolyl, pyridyl, thienyl, pyrazinyl, benzothienyl, benzofuranyl, indolyl, benzimidazolyl, quinolyl or quinazolinyl group, those groups being unsubstituted or substituted by one or more identical or different groups selected from hydroxy, alkoxy, alkyl, amino, alkylamino, dialkylamino, nitro, cyano, polyhaloalkyl, formyl, carboxy, amido, and halogen atoms,
their enantiomers and diastereoisomers, and addition salts thereof with a pharmaceutically acceptable acid or base.

3. Compounds of formula (I) : 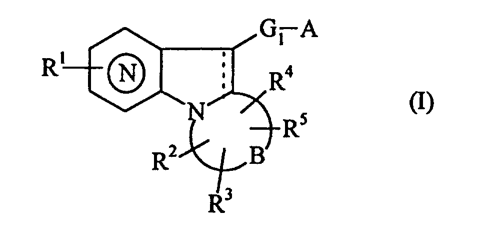 wherein :
- the symbol 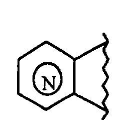 represents the grouping 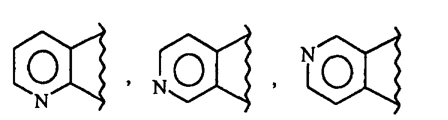 or 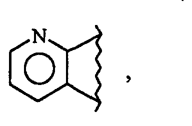
- R¹ represents a halogen atom or a group -R, -OR, -S(O)ₙR, -NRR', 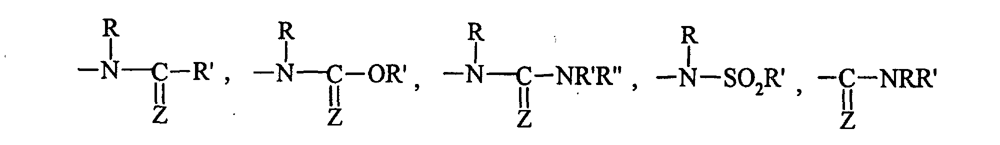 or -SO₂NRR' (wherein n is 0, 1 or 2, Z represents a sulphur or oxygen atom and R, R' and R", which may be identical or different, represent a hydrogen atom or an unsubstituted or substituted linear or branched (C₁-C₆)alkyl group, an unsubstituted or substituted linear or branched (C₂-C₆)alkenyl group, an unsubstituted or substituted linear or branched (C₂-C₆)alkynyl group, an unsubstituted or substituted (C₃-C₈)cycloalkyl group, an unsubstituted or substituted (C₃-C₈)cycloalkyl-(C₁-C₆)alkyl group in which the alkyl moiety is linear or branched, an aryl group, an aryl-(C₁-C₆)alkyl group in which the alkyl moiety is linear or branched, a heteroaryl group or a heteroaryl-(C₁-C₆)alkyl group in which the alkyl moiety is linear or branched,
it also being possible for (R and R') and (R' and R") to form together with the nitrogen atom carrying them a morpholinyl, piperidinyl, piperazinyl or pyrrolidinyl group, those groups being unsubstituted or substituted,
- G₁ represents an alkylene chain having from 1 to 4 carbon atoms wherein one of the CH₂ groups may be replaced by a (C₃-C₈)cycloalkylene group,
- A represents a group 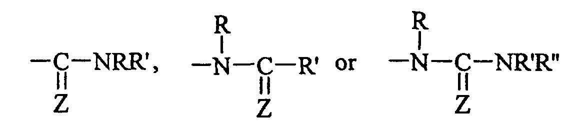 wherein R, R', R" and Z are as defined hereinbefore,
- B forms with the nitrogen atom and the carbon atom to which it is attached a ring having from 5 to 8 ring members, which may contain one or more unsaturated bonds and may contain, in addition to the nitrogen atom, an additional hetero atom selected from oxygen, sulphur and nitrogen,
- R² and R³, which may be identical or different, represent a hydrogen atom or a linear or branched (C₁-C₆)alkyl group, a linear or branched (C₁-C₆)alkoxy group or a hydroxy group,
or R² and R³ together form an oxo group,
- R⁴ and R⁵ represent a hydrogen atom or form together with two adjacent atoms of the B ring carrying them a group selected from aryl and heteroaryl,
- the symbol ----- means that the bond may be single or double, with the proviso that the valence of the atoms is respected,
it being understood that :
- the term "substituted" applied to the terms "alkyl", "alkenyl", "alkynyl", "cycloalkyl", "morpholinyl", "piperidinyl", "piperazinyl" and "pyrrolidinyl" means that those groups may be substituted by one or more groups selected from hydroxy, oxo, alkoxy, alkyl, polyhaloalkyl, and halogen atoms,
- the term "substituted" applied to the term "cycloalkylalkyl" means that the cyclic moiety of the group is substituted by one or more groups selected from hydroxy, oxo, alkoxy, alkyl, polyhaloalkyl, and halogen atoms,
- "aryl" is understood to mean a phenyl, naphthyl or biphenyl group, those groups being unsubstituted or substituted by one or more identical or different groups selected from hydroxy, alkoxy, alkyl, amino, alkylamino, dialkylamino, nitro, cyano, polyhaloalkyl, formyl, carboxy, alkoxycarbonyl, amido, and halogen atoms,
- "heteroaryl" is understood to mean a furyl, pyrrolyl, imidazolyl, pyridyl, thienyl, pyrazinyl, benzothienyl, benzofuranyl, indolyl, benzimidazolyl, quinolyl or quinazolinyl group, those groups being unsubstituted or substituted by one or more identical or different groups selected from hydroxy, alkoxy, alkyl, amino, alkylamino, dialkylamino, nitro, cyano, polyhaloalkyl, formyl, carboxy, amido, and halogen atoms,
their enantiomers and diastereoisomers, and addition salts thereof with a pharmaceutically acceptable acid or base.

4. Compounds of formula (I) according to claim 1 wherein B forms with the nitrogen atom and the carbon atom to which it is attached a pyrrolidine, piperidine, (perhydro)azepine or (perhydro)azocine ring, their enantiomers and diastereoisomers, and addition salts thereof with a pharmaceutically acceptable acid or base.

5. Compounds of formula (I) according to claim 1 wherein B forms with the nitrogen atom and the carbon atom to which it is attached a 1,3-(perhydro)oxazine ring, their enantiomers and diastereoisomers, and addition salts thereof with a pharmaceutically acceptable acid or base.

6. Compounds of formula (I) according to claim 1 wherein R², R³, R⁴ and R⁵ simultaneously represent a hydrogen atom, their enantiomers and diastereoisomers, and addition salts thereof with a pharmaceutically acceptable acid or base.

7. Compounds of formula (I) according to claim 1 wherein R² and R³ represent a hydrogen atom and R⁴ and R⁵ form together with the adjacent carbon atoms carrying them a phenyl group, their enantiomers and diastereoisomers, and addition salts thereof with a pharmaceutically acceptable acid or base.

8. Compounds of formula (I) according to claim 1 wherein R¹ represents a group OR, their enantiomers and diastereoisomers, and addition salts thereof with a pharmaceutically acceptable acid or base.

9. Compounds of formula (I) according to claim 1 wherein G₁ represents a group (CH₂)ₚ wherein p is 2 or 3, their enantiomers and diastereoisomers, and addition salts thereof with a pharmaceutically acceptable acid or base.

10. Compounds of formula (I) according to claim 1 wherein A represents a group NHCOR, their enantiomers and diastereoisomers, and addition salts thereof with a pharmaceutically acceptable acid or base.

11. Compounds of formula (I) according to claim 1 wherein A represents a group CONHR, their enantiomers and diastereoisomers, and addition salts thereof with a pharmaceutically acceptable acid or base.

12. Compounds of formula (I) according to claim 1 that are *N*-[2-(2-methoxy-6*H*-pyrido[2',3':4,5]pyrrolo[2,1-*a*]isoindol-11-yl)ethyl]acetamide, *N*-[2-(2-methoxy-6*H*-pyrido[2',3':4,5]pyrrolo[2,1-*a*]isoindol-11-yl)ethyl)-2-furamide, *N*-[2-(2-methoxy-6*H*-pyrido[2',3':4,5]pyrrolo[2,1-*a*]isoindol-11-yl)ethyl]benzamide, 2-iodo-*N*-[2-(2-methoxy-6*H*-pyrido[2',3':4,5]pyrrolo[2,1-*a*]isoindol-11-yl)ethyl]benzamide and addition salts thereof with a pharmaceutically acceptable acid or base.

13. Compounds of formula (I) according to claim 1 that are *N*-[2-(3-methoxy-6,7,8,9-tetrahydropyrido[3,2-*b*]indolizin-5-yl)ethyl]acetamide, *N*-[2-(2-methoxy-6,7,8,9-tetrahydropyrido[2,3-*b*]indolizin-10-yl)ethyl]-2-furamide, *N*-[2-(2-methoxy-6,7,8,9-tetrahydropyrido[2,3-*b*]indolizin-10-yl)ethyl]acetamide, *N*-[2-(2-hydroxy-6,7,8,9-tetrahydropyrido[2,3-*b*]indolizin-10-yl)ethyl]-2-furamide and addition salts thereof with a pharmaceutically acceptable acid or base.

14. Compounds of formula (I) according to claim 1 that are *N*-[2-(8-methoxy-3,4-dihydro-2*H*-pyrido[2',3':4,5]pyrrolo[2,1-*b*][1,3]oxazin-10-yl)ethyl]acetamide, *N*-[2-(8-methoxy-3,4-dihydro-2*H*-pyrido[2',3':4,5]pyrrolo[2,1-*b*][1,3]oxazin-10-yl)ethyl]-2-furamide and addition salts thereof with a pharmaceutically acceptable acid or base.

15. Compound of formula (I) according to claim 1 that is *N*-[2-(10-methoxy-5,6-dihydro-pyrido[2',3':4,5]pyrrolo[2,1-*a*]isoquinolin-12-yl)ethyl]-2-furamide and addition salts thereof with a pharmaceutically acceptable acid or base.

16. Compounds of formula (I) according to claim 1 that are *N*-[2-(11-methoxy-6,7-dihydro-5*H*-pyrido[2',3':4,5]pyrrolo[2,1-*a*][2]benzazepin-13-yl)ethyl]acetamide, *N*-[2-(11-methoxy-6,7-dihydro-5*H*-pyrido[2',3':4,5]pyrrolo[2,1-*a*][2]benzazepin-13-yl)-ethyl]-2-furamide, *N*-[2-(11-hydroxy-6,7-dihydro-5*H*-pyrido[2',3':4,5]pyrrolo[2,1-*a*]-[2]benzazepin-13-yl)ethyl]-2-furamide and addition salts thereof with a pharmaceutically acceptable acid or base.

17. Compound of formula (I) according to claim 1 that is *N*-[2-(2-methoxy-6-oxo-6*H*-pyrido[2',3':4,5]pyrrolo[2,1-*a*]isoindol-11-yl)ethyl]-2-furamide and addition salts thereof with a pharmaceutically acceptable acid or base.

18. Process for the preparation of compounds of formula (I) according to claim 1, **characterised in that** there is used as starting material a compound of formula (II) : 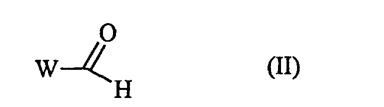 wherein W represents a radical of formula (III) : 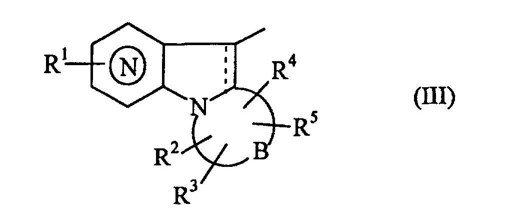 wherein R¹, R², R³, R⁴, R⁵, B, the symbol 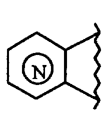 and the symbol ---- are as defined hereinbefore,
which is subjected to
- the action of a reducing agent to obtain a compound of formula (IV) :
W-CH₂OH (IV)
wherein W is as defined hereinbefore,
which, after conversion to the corresponding halogen compound, is subjected to the action of a cyanide salt to yield a compound of formula (V) :
W-CH₂-CN (V)
wherein W is as defined hereinbefore,
- or, in succession, a Wittig reaction and then reduction to obtain a compound of formula (VI) :
W-G'₁-X (VI)
wherein W is as defined hereinbefore, X represents a group CN or COOalk (wherein alk represents an alkyl group) and G'₁ represents a chain as defined hereinbefore for G₁ having from 2 to 4 carbon atoms,
which compounds of formulae (V) and (VI) are hydrolysed in an acidic or basic medium to obtain a compound of formula (VII) :
W-G₁-COOH (VII)
wherein W and G₁ are as defined hereinbefore,
which is subjected, in the presence of a coupling agent or after conversion to the corresponding acid chloride, to the action of an amine HNRR' wherein R and R' are as defined hereinbefore to yield a compound of formula (I/a), a particular case of the compounds of formula (I) : 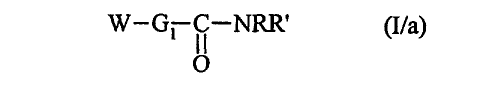 wherein W, G₁, R and R' are as defined hereinbefore,
which compound of formula (I/a), when R and R' simultaneously represent a hydrogen atom, is subjected to the action of NaOBr to yield, after hydrolysis, a compound of formula (VIII) :
W-G₁-NH₂ (VIII)
wherein W and G₁ are as defined hereinbefore,
which is subjected to the action of:
- an acyl chloride of formula (IX) : 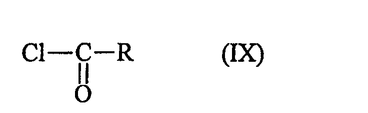 wherein R is as defined hereinbefore, or the corresponding acid anhydride (mixed or symmetric),
to obtain a compound of formula (I/b), a particular case of the compounds of formula (I) : 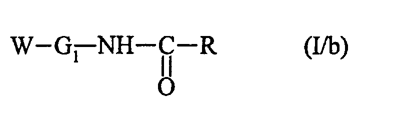 wherein W, G₁ and R are as defined hereinbefore,
which compound of formula (I/b) may also be obtained, when G₁ represents a chain (CH₂)₂, starting from a compound of formula (II) by the action of nitromethane to yield a compound of formula (III') : 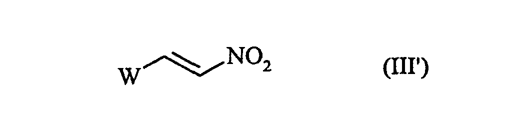 wherein W is as defined hereinbefore,
which is subjected to the action of a reducing agent, such as NaBH₄, for example, to obtain a compound of formula (IV'): 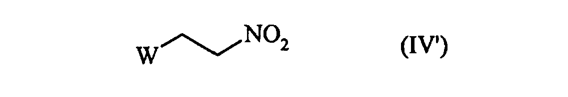 wherein W is as defined hereinbefore,
which is subjected to catalytic hydrogenation to obtain a compound of formula (V') : 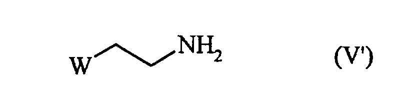 wherein W is as defined hereinbefore,
which is subjected to the action of an acid chloride of formula (IX) or the corresponding acid anhydride (mixed or symmetric) to obtain a compound of formula (I/b'), a particular case of the compounds of formula (I/b) : 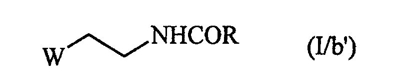 wherein W and R are as defined hereinbefore,
- or which compound of formula (VIII) is subjected to the action of a compound of formula (X) :
O=C=N-R (X)
wherein R is as defined hereinbefore,
to yield a compound of formula (I/c), a particular case of the compounds of formula (I) : 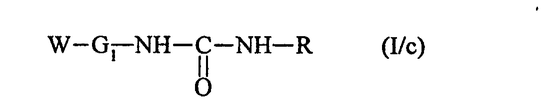 wherein W, G₁ and R are as defined hereinbefore,
which compounds of formulae (I/b) and (I/c) may be subjected to the action of a compound of formula (XI) :
Rₐ-J (XI)
wherein Rₐ can have any of the meanings of R with the exception of the hydrogen atom and J represents a leaving group, such as a halogen atom or a tosyl group,
to yield a compound of formula (I/d), a particular case of the compounds of formula (I) : 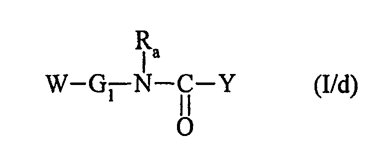 wherein W, G₁ and Rₐ are as defined hereinbefore and Y represents a group R or -NRR' wherein R and R' are as defined hereinbefore,
and/or which compounds of formulae (I/a), (I/b), (I/c) and (I/d) may be subjected to the action of a thionisation agent, such as Lawesson's reagent, to yield a compound of formula (I/e), a particular case of the compounds of formula (I) :
W-G₁-T (I/e)
wherein W and G₁ are as defined hereinbefore and T represents a group 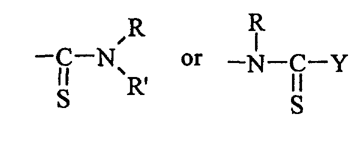 wherein R, R' and Y are as defined hereinbefore,
which compounds (I/a) to (I/e) constitute the totality of the compounds of formula (I) and may be purified according to a conventional separation technique, are converted, if desired, into their addition salts with a pharmaceutically acceptable acid or base, and separated, where appropriate, into their isomers according to a conventional separation technique.

19. Compounds of formula (XVII'): 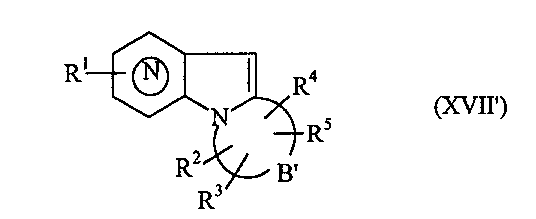 wherein R¹, R², R³, R⁴, R⁵ and the symbol 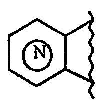 are as defined in claim 1 and B' represents a grouping B as defined in claim 1 except for the piperazinyl group, for use as intermediates in the synthesis of compounds of formula (I).

20. Pharmaceutical compositions comprising as active ingredient at least one compound of formula (I) according to any one of claims 1 to 17 or an addition salt thereof with a pharmaceutically acceptable acid or base, in combination with one or more pharmaceutically acceptable excipients.

21. Pharmaceutical compositions according to claim 20 for use in the manufacture of a medicament for the treatment of disorders of the melatoninergic system.
